# EUROPEAN PATENT SPECIFICATION

(11) **EP 1 478 750 B1**
(45) Date of publication and mention of the grant of the patent: **08.08.2012**
(21) Application number: 00983755.0
(22) Date of filing: 22.11.2000
(51) Int. Cl.: C12N 15/11, C12N 15/63, C07H 21/02, A61K 48/00, C12N 15/67, C12N 15/85

(54) **GENE EXPRESSION BY POSITIVE FEEDBACK ACTIVATION OF A CELL TYPE-SPECIFIC PROMOTER**
GENEXPRESSION DURCH POSITIVE RÜCKKOPPLUNGSAKTIVIERUNG EINES ZELLTYPSPEZIFISCHEN PROMOTORS
EXPRESSION GENETIQUE PAR ACTIVATION A RETROACTION POSITIVE D'UN PROMOTEUR SPECIFIQUE AU TYPE DE CELLULE

(30) Priority: 23.11.1999 US 167085 P
(43) Date of publication of application: 24.11.2004
(73) Proprietor: MAYO FOUNDATION FOR MEDICAL EDUCATION AND RESEARCH, Rochester, MN 55906 (US)
(72) Inventor: VILE, Richard, Rochester, MN 55902 (US); GOUGH, Michael, Rochester, MN 55904 (US)
(74) Representative: Gassner, Wolfgang
(86) International application number: PCT/US2000/032203
(87) International publication number: WO 2001/038488

(56) References cited:
- EP-A- 0 848 061
- WO-A-97/35992
- WO-A-97/38117
- WO-A-99/57290
- US-A- 5 756 343
- EMILIUSEN L ET AL: "A transcriptional feedback loop for tissue-specific expression of highly cytotoxic genes which incorporates an immunostimulatory component" GENE THERAPY, vol. 8, no. 13, July 2001 (2001-07), pages 987-998, XP002336107 ISSN: 0969-7128
- BATEMAN ANDREW ET AL: "Gene delivery of fusogenic membrane glycoproteins to generate local and immune mediated tumor rejection" CANCER GENE THERAPY, vol. 7, no. 12, December 2000 (2000-12), pages S24-S25, XP002336108 & NINTH INTERNATIONAL CONFERENCE ON GENE THERAPY OF CANCER; SAN DIEGO, CALIFORNIA, USA; DECEMBER 07-09, 2000 ISSN: 0929-1903
- MIVECHI N.F. ET AL.: 'Stable overexpression of human HSF-1 in murine cells suggests activation rather than expression of HSF-1 to be the key regulatory step in the heat shock gene expression' J. CELL. BIOCHEM. vol. 59, no. 2, October 1995, pages 266 - 280, XP000570421
- MIYAZAKI M. ET AL.: 'Activation of human multi-drug resistance-1 gene promoter in response to heat shock stress' BIOCHEM. BIOPHYS. RES. COMM. vol. 187, no. 2, September 1992, pages 677 - 684, XP002959563
- CAO G. ET AL.: 'A safe, effective in vivo gene therapy for melanoma using tyrosinase promoter-driven cytosine deaminase gene' VIVO vol. 13, no. 2, March 1999 - April 1999, page 181, XP002958234
- PARK B.J. ET AL.: 'Augmentation of melanoma-specific gene expression using a tandem melanocyte-specific enhancer results in increased cytotoxicity of the purine nucleoside phosphorylase gene in melanoma' HUMAN GENE THERAPY vol. 10, no. 6, April 1999, pages 889 - 898, XP000940967

## Description

### Related Applications

This application claims priority under 35 U.S.C. § 119(e) to U.S. Provisional Application Serial No. 60/167,085, filed November 23, 1999.

### Field of the Invention

The invention is related to the area of gene therapy. In particular, the invention is related to vectors comprising cell type-specific promoter elements that selectively express cytotoxic genes in tumor cells.

### Background of the Invention

A major unresolved problem in the field of gene therapy is how to achieve the expression of a therapeutic gene in target cells where its effects are desired while avoiding its expression in non-target cells. The problem is especially acute when the transgene has the capacity to harm non-tumor cells and tissues, for example, where a suicide gene is used to destroy a tumor. Two basic approaches have been attempted: the transgene can be delivered in the form of a vector targeted specifically to certain types of cells (vector targeting; see, e.g., Peng and Russell, Cur. Opin. Biotech. 10: 454-457 (1999)) or the transgene can be cloned downstream of a cell type-specific promoter (transcriptional targeting; see Vile, et al, Mol. Med. Today 4: 84-92 (1998)). Targeting of vectors can also rely on physically administering them to a particular anatomical location, either by relying on the natural tropisms of the vectors or by engineering them to recognize a molecular target. A combination of these approaches offers the best hope for the systemic delivery of vectors to treat human disease. Vile, et al., *supra.*

A variety of cell type-specific promoters are known. Examples include promoters for tyrosinase (specific for melanoma cells and melanoctyes; see, Bentley, et al, Mol. Cell. Biol. 14: 7996-8006 (1994)), carcinoembryonic antigen (CEA, specific for colorectal cancer cells; see, e.g., Schrewe, et al., Mol. Cell. Biol. 10: 2738-2748 (1990)), alpha fetoprotein (specific for hepatocytes; see, e.g., Ghebranious, et al., Mol. Reprod. Dev. 42: 1-6 (1995)), erb-B2 (specific for breast cancer cells; see, e.g., Pandha, et al., J. Clin. Oncol. 17: 2180 (1999)) and myelin basic protein (specific for glioma cells; see, e.g., Shinoura, et al., Cancer Res. 59: 5521-5528 (1999)).

However, the use of cell type-specific promoters to induce the expression of cytotoxic agents in tumor cells is particularly problematic. The higher the potency of the suicide gene applied (e.g., toxicity), the greater the potential damage to non-tumor cells that receive the gene if the promoter controlling the suicide gene is not perfectly tumor-specific. Further, inadequate promoter specificity can have serious deleterious effects in non-target cells and tissues which are only revealed under certain conditions. For example, previous work has demonstrated that three tandem repeats of an enhancer element from the human tyrosinase gene (the tyrosinase distal element, TDE), when combined with a basal SV40 promoter, is sufficient to support highly selective expression of the cytokine GM-CSF inhuman melanoma cells (Diaz et al. J. Virol. 72: 789-95 (1998)). However, when the TDE-5V40 promoter is used to drive the expression of the envelope glycoprotein from Gibbon Ape Leukemia Virus (GALV), a highly cytotoxic fusogenic membrane glycoprotein (FMG), 3 of 9 non-melanoma cell lines showed significant amounts of cell killing (syncytium formation) after 72-96 hours, indicating that for this construct the TDE-5V40 promoter was not completely cell type-specific. Therefore, the high toxicity of proteins like GALV envelope glycoprotein, which makes them desirable as potent antitumor agents, will result in an unacceptable amount of bystander killing (killing of nearby normal cells) unless the expression of such agents can be made highly specific for their target cells.

While these problems can be overcome by developing promoters with higher specificity, high tissue specificity tends to be achieved at the expense of promoter strength, thereby undercutting the potency of the therapeutic gene.

### Summary Of The Invention

It is an object of the invention to provide materials to selectively express transgenes in a target tissue. It is also an object of the invention to provide materials to amplify the activity of a cell type-specific promoter. It is another object of the invention to provide materials using a cell type-specific promoter that controls expression of a cytotoxic gene, thereby enhancing the selective killing of targeted cells.

The invention further provides a new class of genes with both direct cytotoxic and immunostimulatory properties. It is still another object of the invention to provide materials such as cell type-specific promoters and constructs containing them. A further object of the invention is to provide targeted expression vectors comprising cell type-specific promoters. These and other objects of the invention are provided by one or more of the embodiments described below.

One embodiment of the invention provides a nucleic acid molecule comprising an amplification promoter element (a heat shock element), a cell type-specific promoter, and a cytotoxic gene under control of the cell type-specific promoter. The nucleic acid molecule comprises a sequence encoding a amplification promoter transcription activator ( HSF-1), that activates the amplification promoter element. For some embodiments, when the nucleic acid molecule is expressed in a target tissue, the level of mRNA expression from the construct is at least 100-fold higher or at least 1000-fold higher than if the construct is expressed in a non-target tissue.

Another embodiment of the invention provides a cytotoxic gene under control of the promoter. The promoter is Tyr300.

The invention thus provides new tools and methods for the highly selective expression of transgenes in a desired target tissue.

### Brief Description of the Drawings

The objects and features of the invention can be better understood with reference to the following detailed description and accompanying drawings.

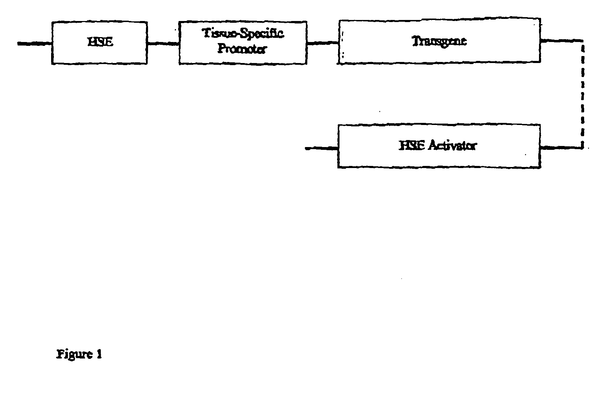

Figure 1 is a schematic diagram of a nucleic acid construct for the selective expression of a transgene in a target cell according to one embodiment of the invention: "HSE" refs to a heat shock element. The dashed line indicates that the sequence encoding a HSE activator can be optionally included in the same construct with the transgene or in a separate construct.

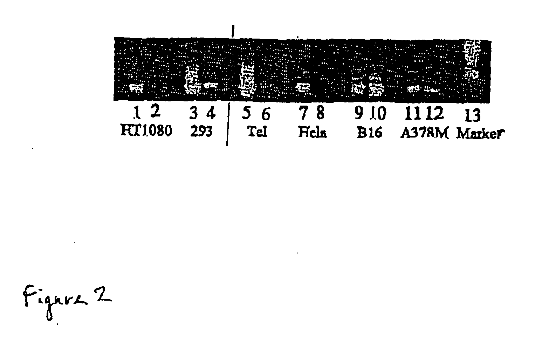

Figure 2 shows the results of RT-PCR to detect expression from CMV promoters (odd numbered lanes) or TDE-SV40 (even numbered lanes; SV40: Simian Virus 40) in non-melanoma cells (HT1080, 293, Tel.CeB6 or Hela cells, as indicated) and melanoma cells (B 16 or A378M). Equal loading was verified with a GAPDH control (not shown).

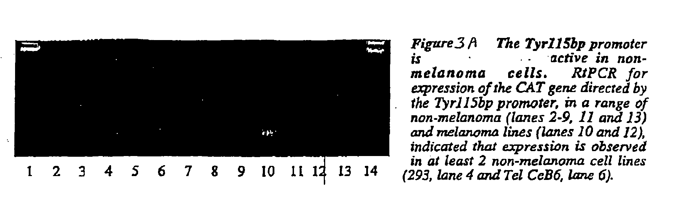

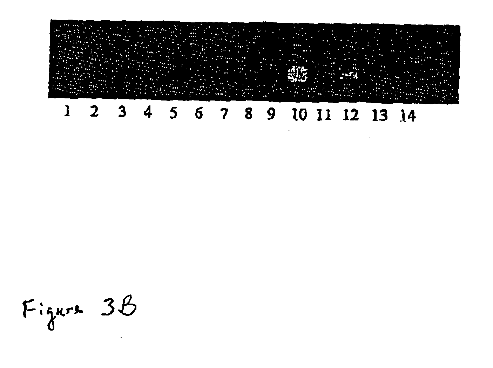

Figures 3A and 3B illustrate the high selectivity of a human tyrosinase 300 base pair (base pair) promoter element using a nestled RT-PCR assay. Figure 3A indicates that the Tyr 115 base pair promoter is not completely inactive in non-melanoma cells. RT-PCR to determine expression of the CAT gene under the control of the Tyr11 base pair promoter was performed using RNA from a range of non-melanoma lines (lanes 2 and 3, HT1080; lanes 4 and 5,293; lanes 12 and 13, A378M). Reverse transcriptase was omitted in the odd numbered lanes. Expression is observed in at least 2 non-melanoma cell lines (293, lane 4 and Tel CeB6, lane 6). Figure 3b shows the same RT-PCR assay of Figure 3A repeated to determine the expression of a CAT gene under the control of the Tyr300 promoter. Lane assignments are the same as those in Figure 3A.

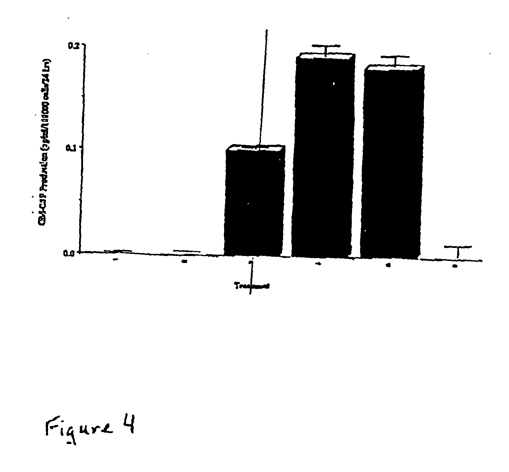

Figure 4 demonstrates that HSE confers heat-shock and mHSF-1 inducibility on the melanoma-specific Tyr 300 base pair promoter. MeWo cells transfected with the Tyr 300-GM-CSF (condition 2) or the HSE-Tyr300-FULL-GM-CSF plasmids (condition 6) express only very low amounts of GM-CSF, demonstrating that Tyr 300 is a very weak promoter. However, transfection of the human melanoma MeWo line with the TDE-SV40-GM-CSF plasmid (condition 3) leads to easily detectable levels of GM-CSF production. In the presence of either heat shock (e.g., 42°C, 30 minutes, condition 4) or a co-transfected mHSF-1 plasmid (condition 5), GM-CSF production is increased significantly following transfection with the HSE-Tyr300-GM-CSF plasmid. Co-transfection of a non-melanoma cell line (HT1080) with the HSE-Tyr 300-GM-CSF plasmid and the HSF-1 cDNA did not yield any detectable GM-CSF production (condition 1).
Figure 4 demonstrates that HSE confers heat-shock and mHSF-1 inducibility on the melanoma-specific Tyr 300 base pair promoter. MeWo cells transfected with the Tyr 300-GM-CSF (condition 2) or the HSE-Tyr 300-FULL-GM-CSF plasmids (condition 6) express only very low amounts of GM-CSF, demonstrating that Tyr 300 is a very weak promoter. However, transfection of the human melanoma MeWo line with the TDE-5V40-GM-CSF plasmid (condition 3) leads to easily detectable levels of GM-CSF production. In the presence of either heat shock (42 °C, 30 minutes; condition 4) or a co-transfected mHSF-1 plasmid (condition 5), GM-CSF production is increased significantly following transfection with the HSE-Tyr 300-GM-CSF plasmid. Co-transfection of a non-melanoma cell line (HT1080) with the HSE-Tyr 300-GM-CSF plasmid and the HSF-1 cDNA did not yield any detectable GM-CSF production (condition 1).

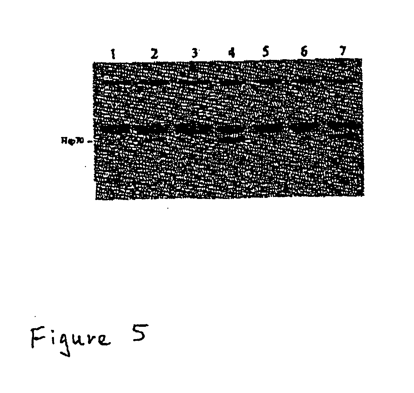

Figure 5 demonstrates hsp70 expression following transient transfection of murine melanoma cells with mutant HSF-1. An immunoblot is shown for Hsp70 expression in B16 cells stably expressing constitutively active human mHSF-1 (deletion 202-316). Lysates of untransfected B-16 cells are shown in lane 1. Lane 2 shows the pooled population of HSF-1 transfected colonies. Lanes 3-7 show clones of individual HSF-1 transfected colonies.

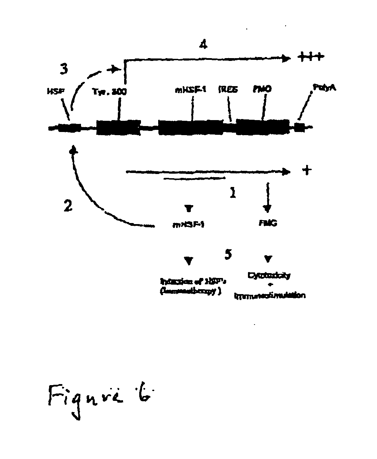

Figure 6 displays a portion of an expression vector in which an HSE transcriptional control element can be used to transactivate gene expression from the melanoma-specific Tyr-300 promoter. (FMG: fusogenic membrane glycoprotein; IRES: internal ribosomal entry site).

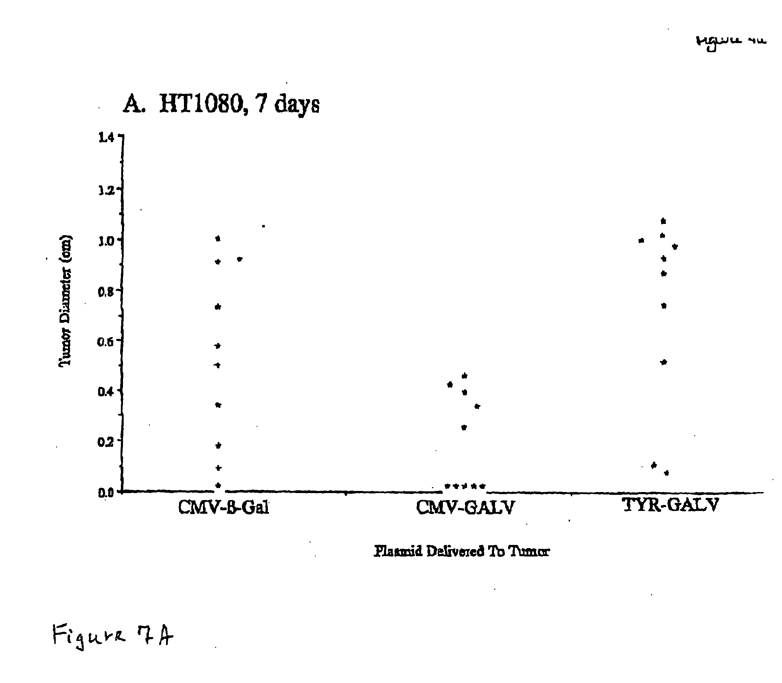

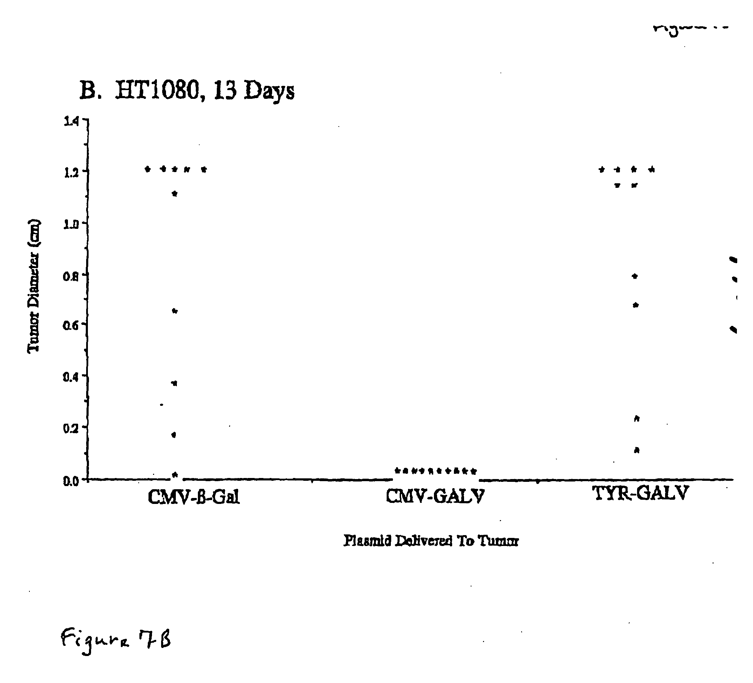

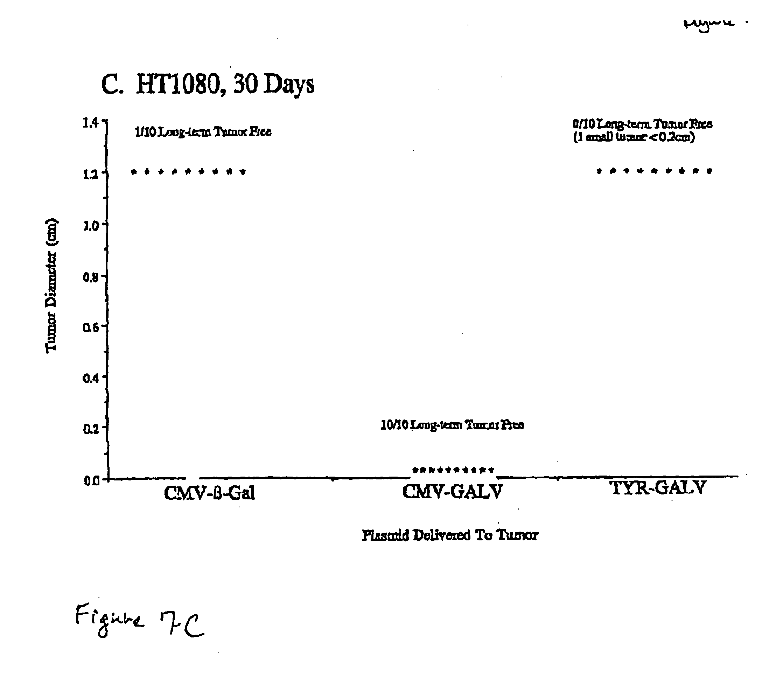

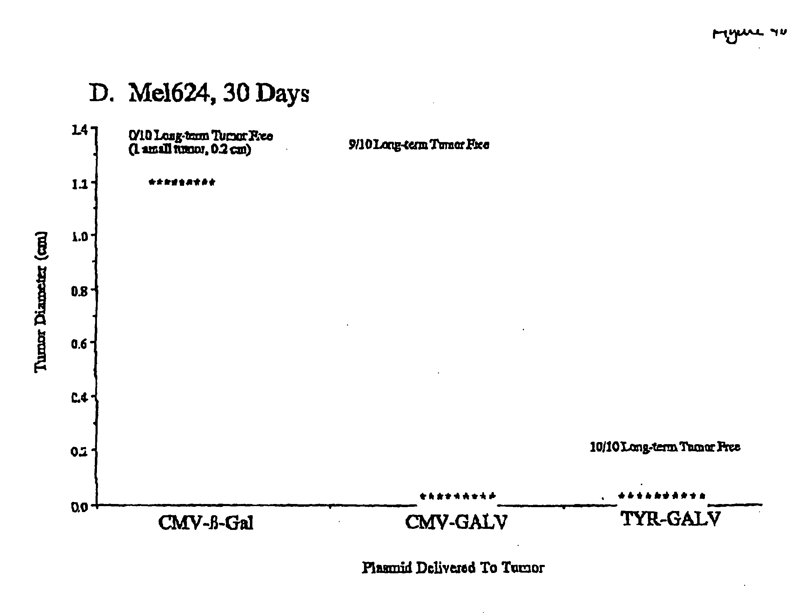

Figures 7A - 7D demonstrate the elimination of primary tumors by plasmids containing cell type-specific promoter elements. See Example 4 for details. Figure 7A. Tumor size is presented seven days following injection of CMV-β-Gal, CMV-GALV, or Tyr300-GALV plasmid DNA (10 ug/tumor) into HT1080 tumors in nude mice. Figure 7B. Same group of mice as in Figure 7A at 13 days post transduction. Figure 7C. Same group of mice as in Figure 7A at 30 days post transduction. Figure 7D. Size of Me1624 tumors (human melanoma) grown in nude mice at 30 days after injection with CMV-β-Gal, CMV-GALV, or Tyr300-GALV plasmids.

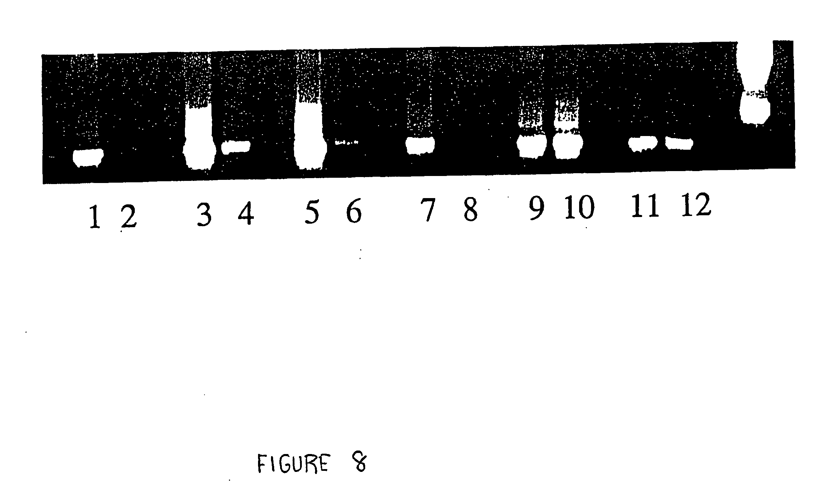

Figure 8 shows the results of RT-PCR experiments to determine expression of a transgene (GALV) from the CMV (odd numbered lanes) or TDE-SV40 (even numbered lanes) promoters in non-melanoma (HT1080, 293, Tel.CeB6 or Hela cells, lanes 1-8) and melanoma cells (B16 and A378M, lanes 9-12). Equal loading was verified with a GAPDH control (data not shown).

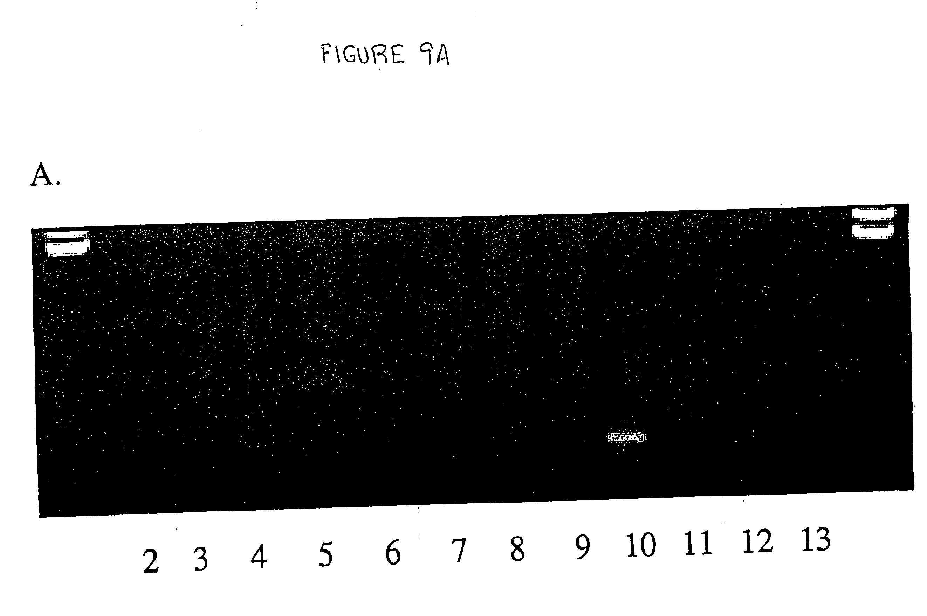

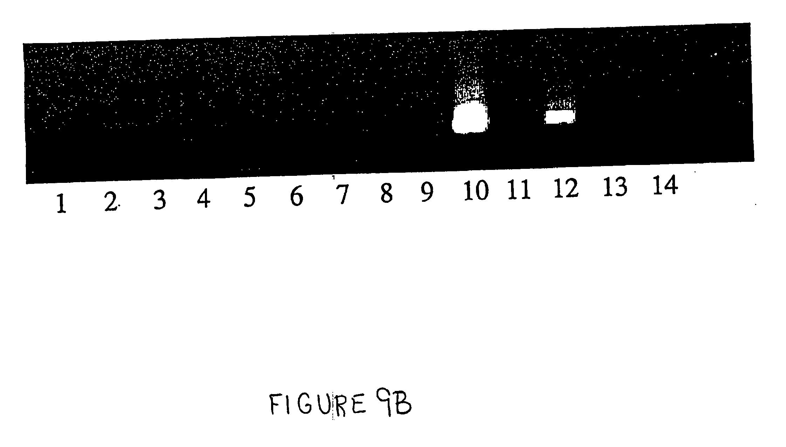

Figures 9A-B illustrate that different elements of the Tyr promoter have different levels of expression in non-melanoma cells. Figure 9A shows the results of RT-PCR performed to determine the expression of the CAT gene directed by the Tyr115 base pair promoter, in a range of non-melanoma (lanes 2-9) and melanoma lines (lanes 10-13). Figure 9B shows the results of a sensitive nested RT-PCR assay to validate the cell type-specific expression of the CAT gene under the control of the Tyr 300 base pair promoter. Lanes are the same as those indicated for Figure 9A. above).

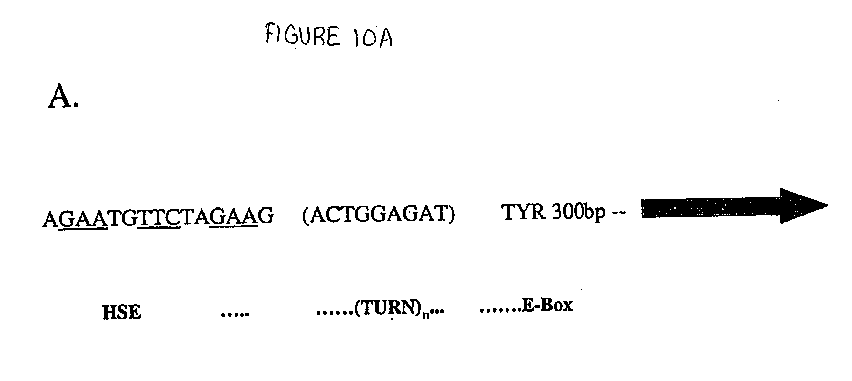

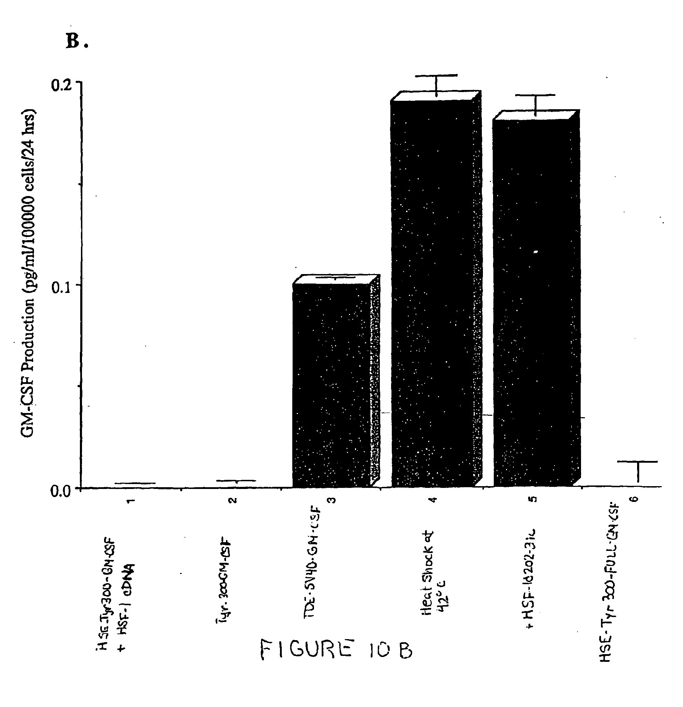

Figures 10A and B illustrate that the HSE element confers heat-shock, and HSF-1d202-316, inducibility on the melanoma-specific Tyr 300 base pair promoter. Figure 10A is a schematic of a nucleic acid construct according to one embodiment of the invention, where the consensus HSE element is cloned upstream of the Tyr-300 promoter element and is separated from the start of the promoter by a linker of either 0 base pair or 10 base pair (i.e., a full turn of the DNA helix) or by 5 base pair (a half turn of the helix). Figure 10B shows the results of a transient transfection assay using the following constructs: Tyr-300-GM-CSF, HSE-Tyr 300-FULL-GM-CSF, TDE-SV40-GM-CSF, HSF-1d202-316 plasmid, and a construct comprising the HSF-1 cDNA.

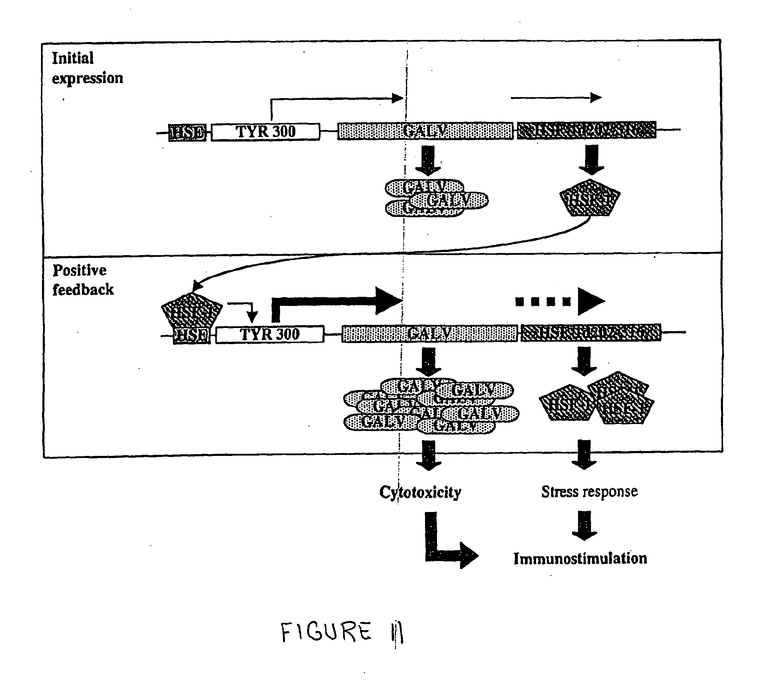

Figure 11 shows a schematic diagram of a positive feedback loop according to one embodiment of the invention.

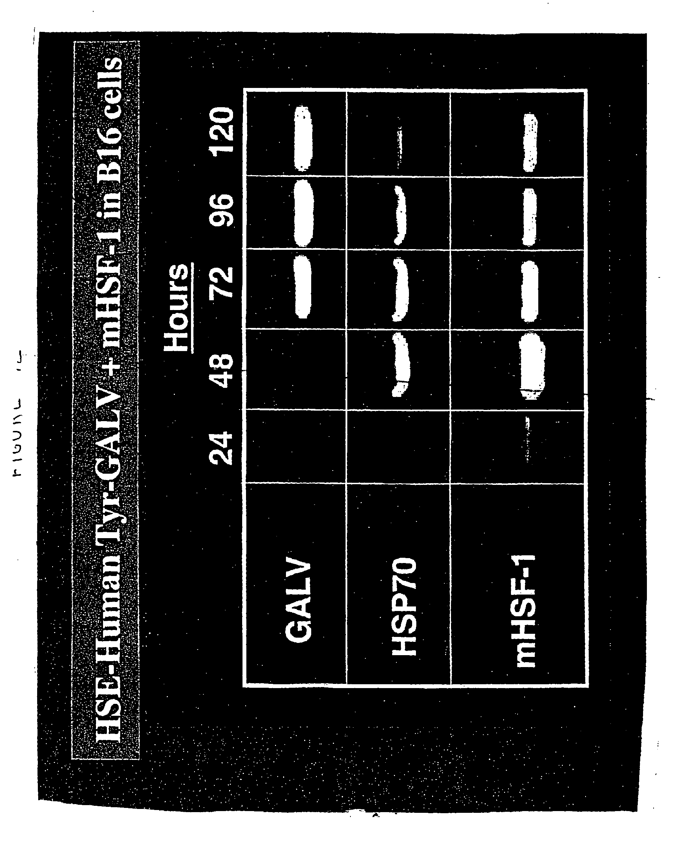

Figure 12 shows that the transcriptional feedback loop according to the invention is operative at the transcriptional level. In one embodiment, 5x10⁵ murine B16 cells, which are not fused by expression of the GALV FMG, were cotransfected with HSE-Tyr-300-GALV (5µg) and mHSF-1(5µg). Cells were harvested at time points following transfection as shown and RT-PCR was used to examine levels of expression of the GALV or HSF-1d202-316 transgenes and of endogenous murine hsp70.

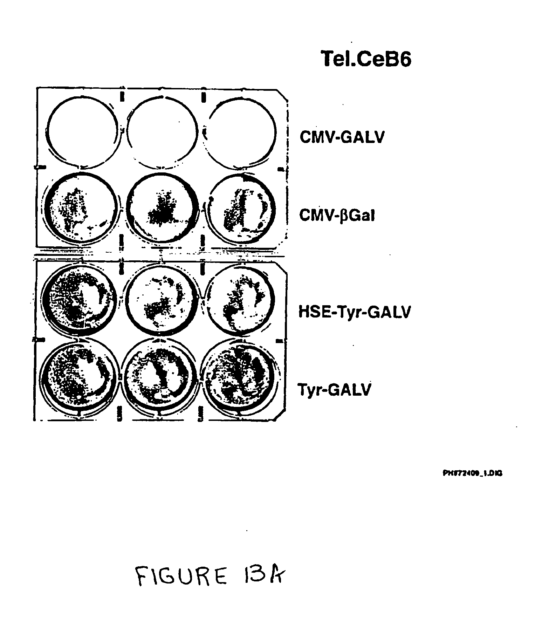

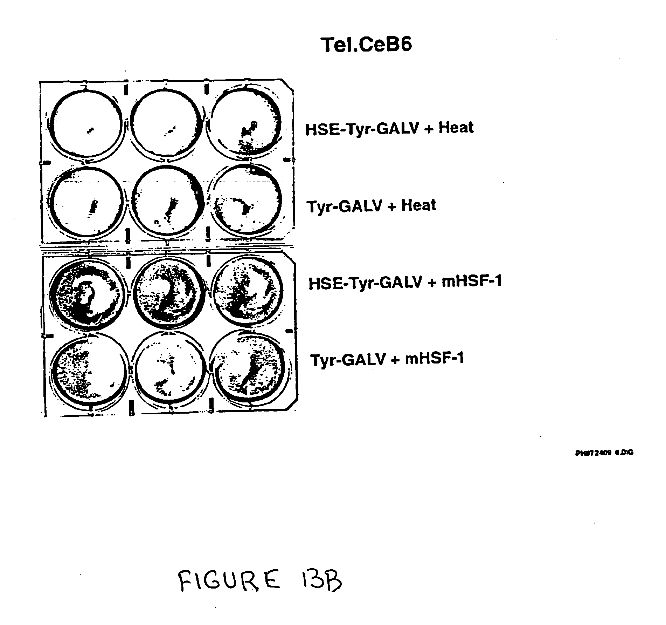

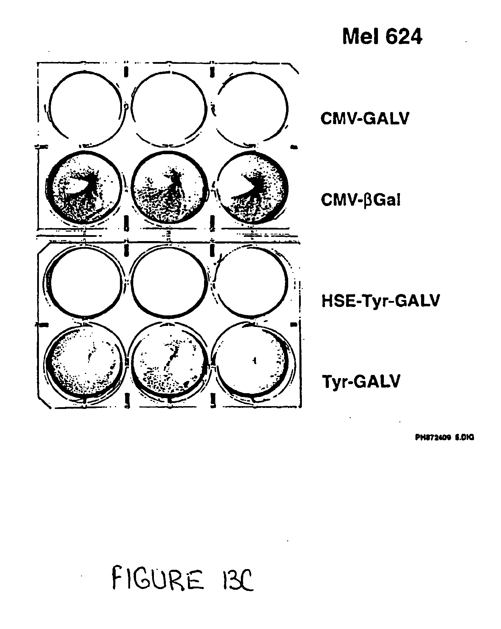

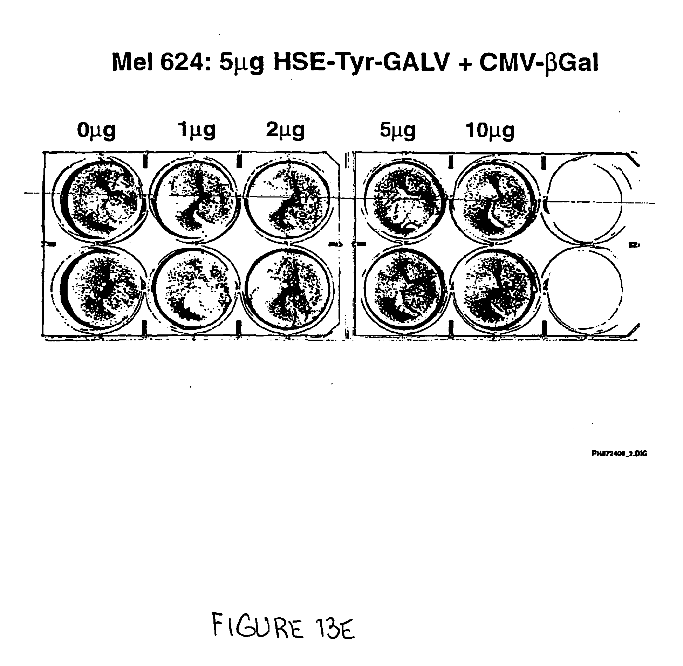

Figures 13A-F illustrate that the HSE-Tyr-300/HSF-1 feedback loop can be used to kill melanoma cells specifically and efficiently. Figures 13A and 13B show the effects of control (calcium phosphate only) transfections and transfection with CMV-GALV of non-melanoma TelCeB6 cells. Figures 13C-D show the effects of transfections of Me1624 cells with the HSE-Tyr-300 and Tyr-300-GALV constructs. gave low levels of toxicity when transfected into a melanoma line, (or MeWo, not shown). Figures 13E-F show the effects of transfection with increasing amounts of co-transfected HSF-1d202-316 β-Gal plasmid.

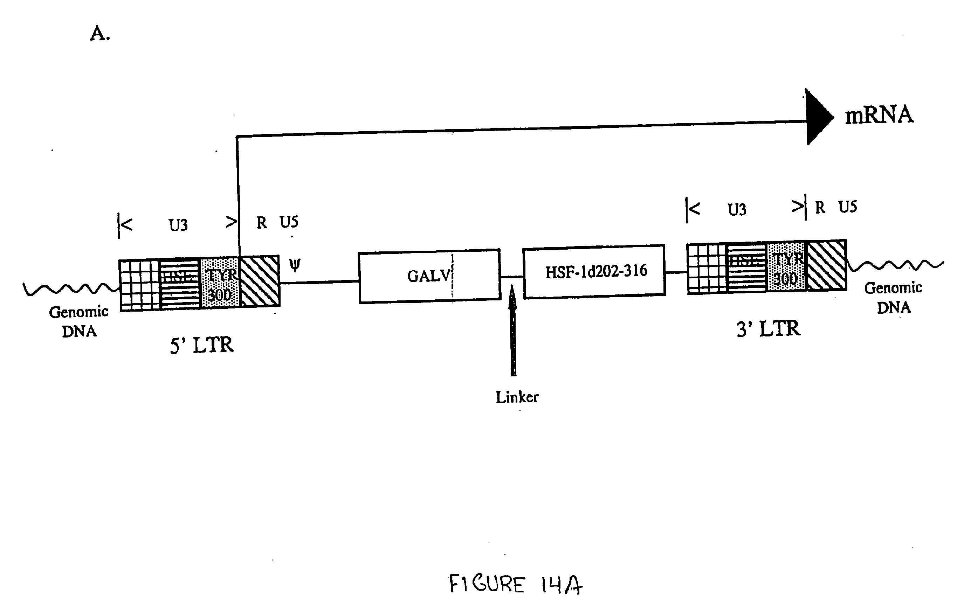

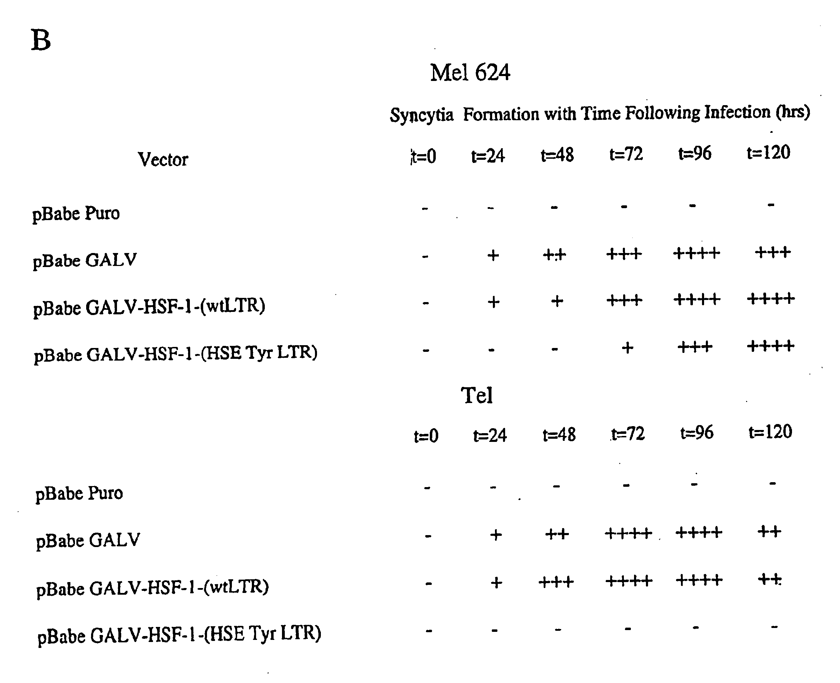

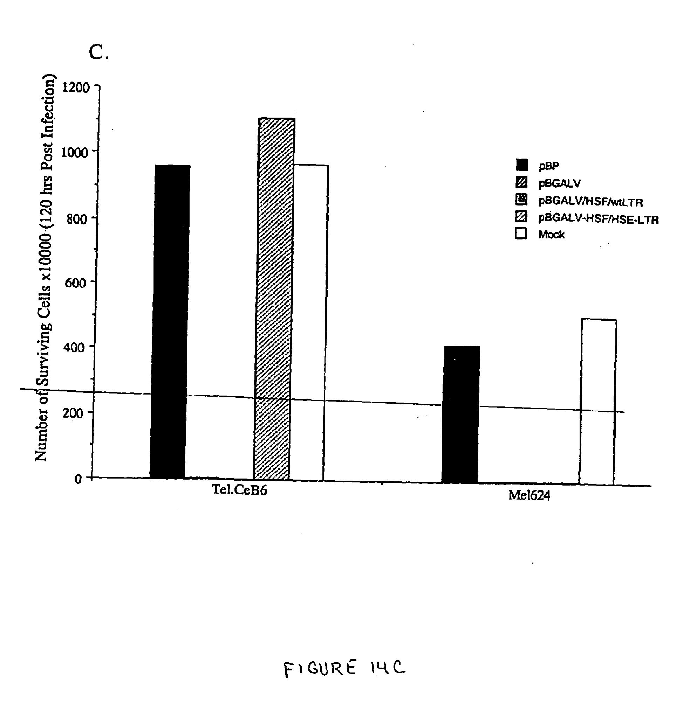

Figures 14A-C show the effects of different vectors on cell type-specific toxicity of a suicide gene in transfected cells, in this embodiment, a cytotoxic fusogenic protein (GALV). Figure 14A is a schematic diagram showing the construction of the pBabe-GALV-HSF-1 (HSE-Tyr LTR) vector, pBabe Puro (no GALV nor HSF-1 cDNAs); pBabe-GALV-HSF-1 (wtLTR), pBabe-GALV-HSF-1 (HSE-Tyr LTR), and pBabe-GALV - pBabe Puro vectors. Figure 14B shows the time course of syncytial development in a melanoma cell line (Mel624) and a non-melanoma cell line (TelCeB6) following infection with viral stocks. The development of syncytia within the cultures was followed at time points following infection (t=0). Syncytia were scored depending upon the proportion of cells in random fields that were within syncytia: -, no visible syncytia; +, 0-20% ; ++, 20-40%; +++40-60%; ++++60-80%; +++++80-100%. Figure 14C shows the cytotoxicity of the viral vectors of Figure 14A. At t=120hrs following infection in the experiment depicted in Figure 14B above, the total number of surviving cells were counted by trypan blue exclusion.

### Description

Tissue specific promoters enable a higher degree of expression of a transgene in certain cells where the transgene product is desired, i.e., in target cells vs. non-target cells. However, in some applications, for example, such as, the delivery of cytotoxic genes to tumor cells, even a low level of expression in non-target cells can be undesirable. The invention provides methods and materials that permit highly selective expression of transgenes in a desired tissue or cell type with little or no expression in non-target cells and tissues. The methods of the invention are useful in the treatment of cancer, genetic disease, and other ailments amenable to gene therapy. The invention is especially useful where selective expression of a transgene in specific target cells with minimal expression in non-target cells is desired for therapeutic or research purposes.

### Definitions

In order to more clearly and concisely describe and point out the subject matter of the claimed invention, the following definitions are provided for specific terms which are used in the following written description and the appended claims.

As used herein, the term "nucleic acid molecule" refers to any natural or synthetic nucleic acid, e.g., DNA, RNA, and chemical analogs and derivatives thereof, either single-stranded, or double-stranded, that is capable of encoding an amino acid sequence and serving as a template for synthesis of a polypeptide. As used herein, the term "nucleic acid molecule" encompasses nucleic acid constructs, cassettes, and vectors.

A "nucleic acid construct," "construct," or "cassette" is a nucleic acid molecule which as been assembled from precursor nucleic acid molecules to form a single nucleic acid molecule with a plurality of functions, e.g., a sequence functioning as a promoter plus a sequence functioning to encode a therapeutic gene. As used herein, the term "construct" encompasses the term "vector."

A "vector" is a nucleic acid molecule which is suitable for introduction of a nucleic acid construct or transgene into a target cell by transfection or transformation. Vectors of the invention include, but are not limited to, any plasmid vector or viral vector known in the art.

A "target cell" is any cell which is the preferred or intended recipient of a nucleic acid construct or transgene which can be delivered either *in vivo* or *ex vivo*, including human cells.

A "transgene" is any nucleic acid sequence that encodes a polypeptide for expression in a target cell.

A "therapeutic transgene" is a transgene encoding a protein which achieves a therapeutic effect when expressed in a cell. A "therapeutic effect" is an effect which ameliorates the symptoms of a disease or which restores molecular parameters to normal levels, i.e., restoring the expression of genes and/or proteins and/or other biomolecules to a level found in individuals who do not have the disease. For example, in one embodiment, the therapeutic effect in a patient with prostate cancer is the restoration of PSA levels to within normal levels (no significant difference determined between levels of PSA in the patient with prostate cancer compared to a patient without prostate cancer, as determined by routine statistical testing to within 95% confidence levels).

A "cytotoxic gene" is any nucleic acid sequence that leads to the death of a cell in which it is expressed within a period of 1, 2, 3, 5, 7, 10, 14, 21, 30, 60, or 100 days.

A "cell type-specific" promoter, promoter element, enhancer, or enhancer element according to the invention is one which leads to a higher degree of expression of a gene under its control in a target cell of the cell type in which the promoter is active than in a non-target cell of cell type in which the promoter is substantially inactive. A promoter, promoter, promoter element, enhancer, or enhancer element, is preferably highly cell-type specific, in which case, the "specificity of expression" of the gene under their control is at least 5-fold, 7-fold, 8-, 9-, 10-, 20-, 30. 50- 100-, 300-, 1000-, 3000-. 10,000-, 30,000-, or 100,000- fold higher in a specific cell type/target cells than in non-target cells. Since non-target cells may differ with respect to expression of a given promoter, promoter element, enhancer, or enhancer element, an average of the expression in at least five different non-target cell types should be compared with the expression in target cells to determine the specificity of expression. A cell type-specific promoter encompasses both tissue-specific promoters and tumor-specific promoters. In one embodiment, the cell-type specific promoter is both tissue-specific and tumor-specific.

As used herein, the terms "promoter element" refers to a subsequence of a promoter that binds to a transcriptional activator. As used herein, the term "promoter element" encompasses enhancer sequences.

An "enhancer" is a sequence which enhances transcription of a promoter and which can be placed in any orientation with respect to the promoter and can function upstream or downstream of a gene, to enhance transcription.

A defined herein, "operably linked" refers to a promoter sequence or promoter element or enhancer which is in sufficient proximity to the transcription start site of a gene to regulate transcription of the gene.

An "amplification promoter element" refers to a sequence, which activates transcription of an operably linked sequence in the presence of an amplification promoter transcription activator. In one embodiment, an amplification promoter element is a heat shock element (HSE) activateable by a "heat shock activator". A heat shock activator is a transcription activator which is expressed or activated when a cell is exposed to heat or other environmental stressors.

A "transcription activator" is a biomolecule (e.g., protein, polypeptide, nucleic acid sequence, and the like) which binds to a promoter element and enhances transcription of an operably linked gene as compared to the transcription of the gene in the absence of the transcription activator.

### Melanoma-Specific Promoters

The invention comprises nucleic acid constructs in which the cell type-specific promoter controlling the expression of the therapeutic transgene is the minimal melanoma-specific promoter of 300 base pair corresponding to bases -300 to -1 of the human tyrosinase gene ("Tyr300"; SEQ ID NO: 1):

(-300) tcatttgcaa ggtcaaatca tcattagttt tgtagtctat

taactgggtt tgcttaggtc aggcattatt attactaacc

ttattgttaa tattctaacc ataagaatta aactattaat

ggtgaataga gtttttcact ttaacatagg cctatcccac

tggtgggata cgagccaatt cgaaagaaaa gtcagtcatg

tgcttttcag aggatgaaag cttaagataa agactaaaag

tgtttgatgc tggaggtggg agtggtatta tataggtctc

agccaagaca tgtgataatc (-1)

Tyr300 is a subsequence of the 5' untranslated region of the human tyrosinase gene whose sequence was previously described by Bentley, et al., Mol. Cell. Biol. 14:7996-8006 (1994). The 300 base pair element of the human tyrosinase promoter contains at least four positive DNA binding elements as well as one negative element as described by Bentley et al , *supra.* Of particular importance is the M box (at -107 to -97), a conserved element found in other melanocyte-specific promoters (Bentley, et al., *supra).*

In one embodiment, Tyr300 is incorporated into a recombinant construct where it functions as a highly selective activator for transgene expression in melanoma cells (see Example 1).

Other melanoma-associated polypeptides are known and their genes can be screened to identify the presence of melanoma-specific promoters, as described above. These polypeptides are described in, for example, Kupsch, et al., Hum. Gene Ther. 9: 737-746 (1998); Neri, et al., J. Invest. Dermatol. 107: 164-170 (1996); and Kirkin, et al., Exp. Clin. Immunogen. 15: 19-32, (1998).

### Amplification of Cell Type-specific Promoter Activity

In selecting for promoter elements with high cell type-specificity, the inventors discovered that the such promoters tend to be weaker than promoters which mediate less selective expression of downstream sequences, i.e., cell type-specificity is achieved at the expense of promoter strength.

According to the invention, a heat shock element is used to amplify the expression of a downstream therapeutic transgene. Heat shock elements ("HSEs") are sequences found within the first 100 base pair 5' of the RNA start site of eucaryotic heat shock genes (see, e.g., Sorger, P. K. Cell 65: 363 (1991). Heat shock genes, such as Hsp70 genes, from different species differ in the number and orientation of HSEs and in the types of other transcription factor-binding sites found upstream. HSEs include the sequence nGAAn, repeated at least two times in head-to-head or tail-to-tail orientation (nGAAnnTTCn or nTTCnnGAAn), and in one embodiment of the invention, the HSE comprises at least two.nGAAn sequences.

An HSE functions in stress-induced promoter activation by binding a positive transactivating factor, the heat shock factor (HSF-1). The binding constant of this factor to the heat shock element is about a hundred-fold higher than that of any other known mammalian transcription factor to its respective binding site, rendering it a very strong promoter element. The present invention provides a strategy to amplify the expression of a transgene by providing at least one upstream HSE, responsive to an HSF. This strategy can be applied to augment the activity of any promoter and consequently boost the expression of any transgene.

HSE is inducible by HSF-1 in the presence of environmental stressors such as heat, anoxia, or ethanol. Therefore, in one embodiment, a target cell is exposed to heat during and/or prior to, and/or after delivery of a therapeutic transgene whose expression is mediated by an HSE element activated by an HSF-1. Heat activates the HSF-1 protein and allows it to enter the nucleus and bind HSE. In one embodiment, "exposing the target cell to heat" comprises elevating the temperature of the target cell, either by localized heating, e.g., that which may be produced by a focused microwave beam, or by generalized heating, e.g., by a temperature bath. In one embodiment of the invention, the production of the therapeutic transgene's product is enhanced by increasing the ambient temperature of the cells to which is has been/ or is being delivered, to a temperature above 37° C. In another embodiment, cells are maintained at a temperature of 38 to 45° C., more preferably 39 to 44° C, and most preferably 40 to 43° C., for a period of 1 to 12 hours, more preferably 4 to 6 hours, and most preferably 6 hours. In another embodiment, the temperature is elevated periodically, i.e., for 1 to 10 hours a day, more preferably 3 to 6 hours a day, for a period of 1 to 21 days or more.

Other suitable temperatures and time periods can be readily determined by one of skill in the art to optimize the efficiency of the HSE, thereby maximizing production levels of a desired therapeutic transgene product. The induction of HSE's in human cells is described further in Ritossa, Experientia (Basel) 18: 571 (1962); Nover, Heat Shock of Eukaryotic Cells Springer, Berlin (1984); Craig, CRC Cit. Rev. Biochem. 18: 239 (1985); Pelham, Trends Genet. 1: 31 (1985); Lindquist, Ann. Rev. Biochem. 55: 1151 (1986); Pelham, et al., EMBO J., 1: 1473 (1982); Mirault, et al., EMBO J. 1: 1279 (1982); Wu, et al., Mol. Cell. Biol. 5: 330 (1985); Voellmy, et al., Proc. Natl. Acad. Sci. USA 82: 4949 (1985); Drabent, et al., Nucl. Acids Res. 14: 8933 (1986); Berger, et al., Somat. Cell. Molec. Genet. 12: 433 (1986); Wu, et al., Proc. Natl. Acad. Sci. USA 83: 629 (1986), for example.

Chemical agents can also be used to induce the HSE, e.g., through their interactions with HSF-1 (see, e.g., as described in U.S. Patent No. 5,137,805. Activators of HSF-1, such as activating antibodies, polypeptides, and peptide fragments, or aptamers, can be introduced by providing their sequences along with those of the therapeutic transgene and the HSF-1, or by administering the activator directly to the patient (e.g., intravenously, intramuscularly, subcutaneously, enterally, or parenterally), by continuous infusion, or by single or multiple boluses. This is preferred when the activator is a drug rather than a polypeptide. The activation of the heat shock element by any of the stressors described above provides synergistic immunotherapeutic effects caused by the induction of heat shock proteins (encoded by sequences which also comprise HSE's), which are stimulated by the rise in HSF-1.

Alternatively, a sequence encoding a constitutively active mutant of HSF-1 can be employed (see Example 2), in which case no heat activation step is required. This embodiment may be used where delivery of the transgenes is performed *in vivo* rather than *ex vivo.*

In one embodiment, an HSE sequence is positioned upstream (5' of) a cell type-specific promoter element that regulates the expression of the therapeutic transgene. In another embodiment, multiple HSE sequences are provided. In one embodiment, an HSF-1 sequence is provided as part of a separate nucleic acid construct. In this embodiment, the construct comprising the therapeutic transgene and the construct encoding the HSF-1 protein are provided sequentially, either within minutes or hours of each other; while in a preferred embodiment, the construct comprising the therapeutic transgene and the construct encoding the HSF-1 protein are be delivered to a cell simultaneously, e.g., in the same pharmaceutical excipient.

In another embodiment, the therapeutic transgene and the HSF-1 encoding sequence are part of the same nucleic acid construct and the transcription of both genes is controlled by the upstream HSE and a cell type-specific promoter. While the low level of promoter activity from the cell type-specific promoter initially supports only a low level of transcription of both transgenes, eventually HSF-1 accumulates to a level which activates the HSE and thereby increases transcription of both the therapeutic transgene and the HSF-1 encoding gene. This positive feedback loop converts a highly specific, but low level of promoter activity, into a highly specific and strong level of promoter activity in a target cell for which the promoter was designed.

A construct which exemplifies this approach is depicted in Figure 6. In this embodiment, a nucleic acid molecule embodying this method comprises: (1) an HSE sequence such as the human consensus sequence 5'-AGAATGTTCTAGAAG-3' (SEQ ID NO:2, see Zuo, et al., Mol. Cell. Biol. 15: 4319-4330 (1995)); which is placed upstream of (2) any promoter, and which is followed in a 3' direction by (3) a transgene whose expression is desired, which turn is followed in a 3' direction by (4) a sequence that encodes a transcription factor or transcriptional activator that binds to and activates HSE, such as human heat shock factor-1 (HSF-1). The order of the transgene and the sequence encoding the transcription factor is unimportant; either gene can be positioned near the promoter, as long as transcription of both coding sequences is driven by the promoter. Where HSF-1 in encoded by a sequence not under control of HSE, then simple amplification of expression of the transgene under control of HSE will result.

In another embodiment, an internal ribosomal entry site (IRES) is placed between the therapeutic transgene and the HSF-1 encoding sequence, so that both the product of the therapeutic transgene and the HSF-1 protein are translated from the same message. Internal ribosome entry sites (IRES, also called ribosomal landing pads) are sequences that enable a ribosome to attach to mRNA downstream from the 5' cap region and scan for a downstream AUG start codon, for example in polycistronic mRNA. See generally, Miles, et al., U.S. Patent 5,738,985 and N. Sonenberg and K. Meerovitch, Enzyme 44: 278-91 (1990).

Addition of an IRES between the coding sequences for two transgenes, for example, a cytotoxic gene and a gene encoding HSF-1, can enable the independent translation of either the transgene or HSF-1 from a dicistronic or polycistronic transcript.

IRES sequences can be obtained from a number of RNA viruses (e.g., picornaviruses, hepatitis A, B, and C viruses, and influenza viruses) and DNA viruses (e.g., adenovirus). IRES sequences have also been reported in mRNAs from eukaryotic cells (Macejak and Sarnow, Nature 353: 90-94 (1991) and Jackson, Nature 353: 14015 (1991)). Viral IRES sequences are detailed in the following publications: (a) Coxsackievirus: Jenkins, J. Gen. Virol. 68: 1835-1848 (1987); Iizuka, et al., Virology 156: 64-73 (1987); and Hughes, et al., S. Gen. Virol. 70: 2943-2952; (b) Hepatitis A Virus: Cohen, et al., Proc. Natl. Acad. Sci. USA 84: 2497-2501 (1987); and, Paul, et al., Virus Res. 8: 153-171 (1987); (c) Poliovirus: Racaniello and Baltimore, Proc. Natl. Acad. Sci. USA 78: 4887-4891 (1981); and Stanway, et al., Proc. Natl. Acad. Sci. USA 81: 1539-1543 (1984); (d) Rhinovirus: Deuchler et al., Proc. Natl. Acad. Sci. USA 84: 2605-2609 (1984); Leckie, G., Ph.D. thesis, University of Reading, UK; and Skern, et al., Nucleic Acids Res. 13: 2111 (1985); (e) Bovine enterovirus: Earl et al., J. Gen. Virol. 69: 253-263 (1988); (f) Enterovirus type 70, Ryan, M.D. et al., S. Gen. Virol. 71: 2291-99 (1989); (g) Theiler's murine encephalomyelitis virus: Ohara, et al., Virology 164: 245 (1988); and, Peaver, et al., Virology 161: 1507 (1988); (h) Encephalomyocarditis virus: Palmenberg, et al., Nucl. Acids Res. 12: 2969-2985 (1984); and Bae, et al., Virology 170: 282-287 (1989); (i) Hepatitis C Virus: Inchauspe, et al., Proc. Natl. Acad. Sci. USA 88: 10293 (1991); Okamoto, et al., Virology 188: 331-341 (1992); and Kato, et al., Proc. Natl. Acad. Sci. USA 87: 9524-9528 (1990); and (i) Influenza virus: Fiers, W., et al., Supramol. Struct. Cell Biochem. (Suppl 5): 357 (1981).

In other embodiments, other stress inducible promoter elements and stress inducible transcriptional activators, in addition to HSEs and HSFs, respectively, can be used and are encompassed within the scope of the invention. Examples of such types of promoter elements and the activators which regulate them are described in Davis, J. Biol. Chem. 268: 1553 (1993); Holbrook, et al., in: Stress-Inducible Cellular Responses, Feige, U., et al., Eds., Birkhauser Verlag (1996); Datta, et al., Proc. Natl. Acad. Sci. USA, 89(21): 10149-10153 (1992); Datta, et al., Proc. Natl. Acad. Sci. USA, 90(6): 2419-2422, (1993); Alexandropoulos, et al., Nucleic Acids Research, 20(9): 2355-2359 (1992); Attar, et al., Molecular and Cellular Biology, 12 (5): 2432-2443, (1992); S. Qureshi, et al.,; The Journal of Biological Chemistry, 266(17): 10802-10806 (1991); .U.S. Patent No. 6,034,228; U.S. Patent No. 5,827,685; and U.S. Patent 5,770,581, and in U.S. Provisional Application Serial No. 60/193,977, filed March 31, 2000.

As with the HSE-HSF "circuit," the stress-inducible promoter elements described above can be activated by exposing the target cell to the appropriate stressor, which in turn activates the transcription activator, or alternatively, by rendering the expression of the stress-activated transcriptional activator constitutive, e.g., by site-directed or random mutagenesis of the stress activated transcriptional activator and by screening for constitutive activators in cell lines, for example. Amplification promoter elements encompassed generally within the scope of the invention include any promoter element or enhancer sequence responsive to a transcriptional activator which when operably linked to a therapeutic transgene, which in turn is under the control of a highly cell type specific promoter, is capable of driving expression of the transgene at levels suitable for achieving a therapeutic effect.

### Transgenes for Use in the Nucleic Acid Constructs

The nucleic acid molecules, constructs, and vectors of the invention can employ any desired transgene for delivery to, and expression in, a target cell. In one embodiment, the transgene encodes for a protein product whose presence is desired in the target cell for therapeutic, investigational, or other purposes. For example, in one embodiment, the transgene encodes a protein which is defective in, or absent from, the target cell because of genetic disease or a pathological condition.

Genes and the diseases associated with them that are appropriate targets for gene therapy using the methods of the invention include, but are not limited to: AGA aspartylglucosaminidase (GenBank Acc. No. X55330), aspartylglucosaminuria; ALDOB aldolase B, fructose-bisphosphate (GenBank Acc. No. X02747), fructose intolerance; BLM Bloom syndrome (GenBank Acc. No. U39817), Bloom syndrome; CFTR cystic fibrosis transmembrane conductance regulator (GenBank Acc. No. S64699), cystic fibrosis; CLCN1 chloride channel 1, skeletal muscle (GenBank Acc. No. Z25884), Thomsen disease; CRH corticotropin releasing hormone (GenBank Acc. No. V00571), ACTH deficiency; DBH dopamine beta-hydroxylase (GenBank Acc. No. X 13255), Dopamine-beta-hydroxylase deficiency; Fl 1 coagulation factor XI (plasma thromboplastin antecedent) (GenBank Acc. No. M13 142), Factor XI deficiency; GAA glucosidase, alpha, acid (GenBank Acc. No.X55079), glycogen storage disease II; GALC galactosylceramidase (GenBank Acc. No. D86181), Krabbe disease; GALT galactose-1-phosphate uridylyltransferase (GenBank Acc. No. M1873 1), galactosemia; HBB hemoglobin, beta (GenBank Acc. No.V00497), sickle cell anemia (beta-thalassemia); HD huntington (GenBank Acc. No. L12392), Huntington disease; FTL ferritin, light polypeptide (GenBank Acc. No. MI1147), hyperferritinemia-cataract syndrome; MAOA monoamine oxidase A (GenBank Acc. No.M68840), Brunner syndrome; MAT1A methionine adenosyltransferase I, alpha (GenBank Acc. No. D49357), hypermethioninemia; PAH phenylalanine hydroxylase (GenBank Acc. No. U49897), phenylketonuria; PROS 1 protein S (alpha) (GenBank Acc. No. M14338), protein S deficiency; OA1 ocular albinism (GenBank Acc. No. Z48804), Nettleship-Falls type; SGSH N-sulfoglucosamine sulfohydrolase (sulfamidase) (GenBank Acc. No. NM000199), Sanfilippo syndrome, type A; CCND1 cyclin D1 (PRAD1: parathyroid adenomatosis 1) (GenBank Acc. No. X59798); parathyroid adenomatosis 1, centrocytic lymphoma; DMD dystrophin (GenBank Acc. No. X15149), muscular dystrophy, Duchenne.

### Cytotoxic Transgenes Which Provide an Immunostimulatory Component

Gene therapy designed to eradicate tumors has benefited from strategies for simultaneously killing the tumor cells and stimulating the immune response. For example, tumor cell death induced by the HSV thymidine kinase/gancyclovir system also induces hsp70 expression, which in turn induces infiltration of T-cells, macrophages, and dendritic cells, and also increases the expression of cytokines (see, e.g., Todryk, et al., J. Immunol. 163: 1398-1408 (1999)). Infection by replication-competent adenovirus present in adenoviral vectors also induces hsp70 expression and therefore stimulates an immune response to the delivery of cytotoxic transgenes (Melcher, et al., Hum. Gene Ther. 10: 1431-1442 (1999)). Another approach to enhance the immunogenicity of tumors is to provide hsp70 directly to the cells (e.g., by transfecting them with cDNA encoding hsp 70 as described in Melcher, et al., Nat. Med. 4: 581-587 (1998), for example).

In one embodiment, a transgene for use with the invention is a cytotoxic gene or suicide gene that is intended to selectively destroy the target cell. Examples of cytotoxic genes include GAL Venv (e.g., Genbank Acc. No. M26927), HSVTK (e.g., Genbank Acc. Nos. AF057310, X0I712), cytosine deaminase (e.g., Genbank Acc. No. S56903), nitroreductase (e.g., Genbank Acc. No. A23284), or VSV glycoprotein G (e.g., Genbank Acc. No. X03633).

In one embodiment of the invention, a therapeutic transgene according to the invention, encodes a protein with both direct cytotoxic and immunostimulatory properties (e.g., fusogenic proteins) which is cloned downstream of a cell type-specific promoter and an HSE. One example of such a protein is the fusogenic membrane glycoprotein (FMG) (Batman, et al., Cancer Res. 60: 1492-1497 (2000); Diaz, et al. Gene Three. 2000; In press*;* Fielding, et al., Hum Gene Three. 11: 817-826 (2000). FMGs kill tumor cells by causing fusion between cells expressing the FMG and neighboring cells which express the receptor for the FMG.

The fusogenicity of fusogenic proteins means that large local bystander effects can be achieved, where non-expressing cells can be recruited into large multi-nucleated syncytia and eventually killed. Indeed, the bystander killing of an FMG *in vitro* is at least one log higher than that of the conventional suicide genes, HSVtk or CD, in most tumor cell lines tested so far (Bateman, et al., *supra).* In addition, expression of viral FMG is also highly immunostimulatory as judged by the immunogenicity of FMG-expressing vaccines and by the induction of stress related proteins such as inducible heat shock proteins during the killing process (Bateman, et al., *supra,* Diaz, et al., *supra*). These dual properties of high local killing capacity and immunostimulatory activity make fusogenic proteins attractive transgene products for use in gene therapy of cancer.

Fusogenic proteins are known in the art and include, but are not limited to, viral FMGs such as type G membrane glycoproteins of rabies virus, Mokola virus, vesicular stomatitis virus, and Togavirues, as well as murine hepatitis virus JHM surface projection protein, porcine respiratory coronavirus spike glycoprotein, porcine respiratory coronavirus membrane glycoprotein, avian infectious bronchitis spike glycoprotein and its precursor, bovine enteric coranavirus spike protein, paramyxovirus SV5 F protein, Measles virus F protein, canine distemper virus F protein, Newcastle disease virus F protein, human parainfluenza virus 3 F protein, simian virus 41 F protein, Sendai virus F protein, human respiratory syncytial virus F protein, Measles virus hemagglutinin, simian virus 41 F protein, Sendai virus F protein, human respiratory syncytial virus F protein, Measles virus hemagglutinin, simian virus 41 hemagglutinin neuraminidase proteins, human parainfluenza virus type 3 hemagglutinin neuramidase, Newcastle disease virus neuramidase, human herpesvirus 1 gH, simian varicella virus gH, human herpesvirus gB proteins, cercopithecine herpesvirus gB proteins, Friend murine leukemia virus envelope glycoprotein, influenza virus hemagglutinin, poxvirus membrane glycoprotein, Russian Far East encephalitis virus membrane glycoprotein, Venezuelan equine encephalitis virus membrane glycoprotein and varicella virus membrane glycoprotein. FMGs and their sequences are described further in U.S. Provisional Application Serial No. 60/193,977, filed March 31, 2000.

As discussed above, the ability to target gene expression to tumor cells is an essential prerequisite for safe and effective gene therapy of cancer. The more potent the therapeutic gene that is used (i.e., the more toxic), the greater is the need for tight control of its expression to avoid toxicity in non-cancer cells. For example, transcriptional elements which lead to 'phenotypic' tissue specificity of transgene expression with a GM-CSF therapeutic gene (Diaz, et al., J Virol., (1998) 72: 789-795) are insufficient to limit the expression of a much more potent FMG cDNA to the specific cell types in which the GM-CSF gene is expressed.

Therefore, in one embodiment of the invention, an FMG encoding sequence is operably linked to a highly tumor-specific promoter which results in FMG expression greater than 100-fold to 1000-fold in tumor cells compared to non-tumor cells. In one embodiment of the invention, the positive feedback loop described above is used to amplify the tumor specific gene expression of an FMG. In this embodiment, an amplification promoter element, such as an HSE, is cloned upstream of a highly tumor-specific promoter which in turn is cloned upstream of an FMG-encoding sequence. The tumor specific expression of FMG is validated by performing a nested polymerase chain reaction (PCR) protocol, to verify that the FMG gene is silent in all cell types except the specific tumor type corresponding to the tumor-specific promoter. In one embodiment, the expression of the FMG gene is evaluated in at least 6 different cell types (e.g., different types of normal tissues and/or different types of tumors), at least 5 of which must not express FMG.

The positive feedback loop according to the invention has three principal advantages where a gene encoding a cytotoxic protein is used as the therapeutic transgene, and HSE and HSF-1 are used as the amplification promoter element, and amplification promoter element transcription element, respectively: 1) it leads to highly tissue specific expression of a very potent cytotoxic gene; 2) it allows increasing levels of the cytotoxic gene to be expressed which leads to very effective local tumor cell killing; and 3) it also leads to the expression of HSF-1 which transactivates endogenous HSE elements and leads to cell type-specific expression of cellular stress proteins, including hsp70 and natural killer (NK) cell receptors. The induction of heat shock proteins (hsps) is a highly potent immune adjuvant to tumor cell killing and leads to the generation of long term anti-tumor immunity. See, e.g., Melted, et al., Nat Med. 4: 581-587 (1998); Todryk, et al., J Immunol 163: 1398-1408 (1999); Srivastava, et al., Immunity 8: 657-665 (1998).

Therefore, in one embodiment, a construct is provided which comprises a stress-responsive transcriptional activator (e.g., HSF-1) encoding gene, and the expression of the FMG is responsive to a stressor (e.g., heat). However, in a further embodiment, a mutant form of the stress-responsive transcriptional activator which is constitutively active even in the absence of stress (see, Zuo, et al., Mol. Cell. Biol. 15: 4319-4330 (1995), who disclose constitutively active HSF-1 proteins) is cloned downstream of an FMG cDNA. In this embodiment, low levels of expression from the cell type-specific promoter (e.g., tissue-specific and/or tumor-specific promoter) is observed to initiate expression of both FMG and the transcription activator encoding sequences; the expression of both transgenes is amplified as the transcription activator sequences accumulate in the cell.

### Delivery of Nucleic Acid Constructs to Target Tissues

Nucleic acid molecules and constructs providing therapeutic transgenes under the control of highly cell-type specific promoters and amplification promoter elements, can be incorporated into a vector and administered to any mammal, including a human. Many such vectors are commercially available, and other suitable vectors can be readily prepared and obvious to the skilled artisan. The exact design of the vector depends on such factors as the choice of the host cell to be transformed and/or the type of protein desired to be expressed. Suitable vectors can be produced by ligating the desired construct into a plasmid or viral vector suitable for expression in eukaryotic cells (see, for example, Broach, et al., Experimental Manipulation of Gene Expression, ed. M. Inouye (Academic Press, 1983) p. 83; Molecular Cloning: A Laboratory Manual, 2nd Ed., ed. Sambrook, et al. (Cold Spring Harbor Laboratory Press, 1989) Chapters 16 and 17.

Examples of vectors that can be used include, but are not limited to, plasmids such as pBR322, pUC, or Co 1E1; adenovirus; Sindbis virus; simian virus 40; cytomegalovirus; and retroviral vectors such as murine sarcoma virus, mouse mammary tumor virus, Moloney murine leukemia virus, and Rous sarcoma virus. Bacterial vectors can be used, such as Salmonella ssp., Yersinia enterocolitica, Shigella spp., Vibrio cholerae, Mycobacterium strain BCG, and Listeria monocytogenes. Minichromosomes such as MC and MC1, bacteriophages, cosmids (plasmids into which phage lambda cos sites have been inserted) and replicons (genetic elements that are capable of independent extrachromosomal replication).

The vectors described above can additionally comprise sequences encoding one or more selectable markers, including, but not limited to, the gene that encodes dihydrofolate reductase and the genes that confer resistance to neomycin, tetracycline, ampicillin, chloramphenicol, kanamycin and streptomycin resistance. To improve incorporation into the genome of the target cell (if desired), a retroviral vector can be used, and long terminal repeat (LTR) sequences can be added on either side of the expression construct (see, e.g., Vile, et al., Virology 214: 307-313 (1995).

Delivery of a therapeutic transgene under the control of a highly cell-type specific promoter can be by any means known in the art, including oral or intranasal administration; intramuscular, intradermal, intraperitoneal, or subcutaneous injection, including injection using a biological ballistic gun ("gene gun"). Administration of the therapeutic transgene can be repeated at any desired interval as needed to achieve therapeutic efficacy. Additional components can be added to a vector to improve its selective delivery to target cells and to repress its delivery to non-target cells. Examples of approaches that can be used include host range extension, entry enhancement, and host range restriction, as described in Peng and Russell, Cur. Opin. Biotech. 10: 454-457 (1999), reference.

The above disclosure generally describes the present invention. A more complete understanding can be obtained by reference to the following specific examples, which are provided herein for purposes of illustration only and are not intended to limit the scope of the invention.

### Examples

### Plasmids and Cell Lines

For the analysis of melanoma specific gene expression, plasmids and viruses were transduced into either melanoma (MeWo, Me1624, A378M, B16 or 1735) or non-melanoma (HT1080; 293; Vero, Tel.CeB6, HeLa, CMT93) cell lines. The human tyrosinase promoter plasmids (300 base pairs; 115 base pairs or 65 base pairs) are as described by Bentley et al., *supra,* and Diaz, et al, *supra.* The TDE-SVO plasmid consists of three repeated copies of the 20 base pairs Tyrosinase Distal Element (TDE) upstream of the minimal SV40 basal promoter (Promega) (Diaz, et al. J. Virol. (1998) 72: 789-795, the entirety of which is incorporated by reference herein). The cDNA of the mutated HSF-1 transcription factor is described in Zuo, et al., Mol. Cell Biol. 15: 4319-4330, (1995) and consists of a deletion of the wild type HSF-1 cDNA corresponding to amino acid positions 202-316. The HSE element - 5'- AGAATGTTCTAGAAG-3' was synthesized as a consensus sequence which confers heat shock and HSF-1 responsiveness on heterologous genes as described in Amin, et al.; Mol. Cell Biol. 8: 3761-3769 (1988) and Goldenberg, et al., J. Biol. Chem. 263: 19734-19739 (1988).

To assess promoter/enhancer strengths, plasmids were constructed using standard techniques such that different promoters and promoter/enhancer fragments were placed upstream either of the Chloramphenicol Acetyl Transferase (CAT) gene (Diaz, et al, *supra*), the human GM-CSF gene (Wong, et al., Science 228: 810-815 (1985), ) or the cDNA of the Gibbon Ape Leukaemia Virus fusogenic membrane glycoprotein (GALV-FMG) (Bateman, et al. Cancer Res. 60: 1492-1497 (2000); Fielding, et al., *supra*). Levels of gene expression were assayed using either CAT assays (Diaz, et al., *supra),* by measuring levels of human GM-CSF secreted from the plasmids by ELISA (Pharmingen) or by semi-quantitative RT-PCR as described below.

Hybrid LTR retroviral vectors were constructed from the parental Mo-MLV retroviral plasmid pBabePuro (Morgenstern, et al., Nucleic Acids Research 18: 3587-3596 (1990) ). Manipulations to the 3'LTR were made in the plasmid pSKLTR followed by reassembly into the pBabePuro plasmid through Clal-Pvul ligations as described in Diaz, et al., *supra.*

### Analysis of Gene Expression from Cells by Reverse Transcriptase Polymerase Chain Reaction

RNA was prepared from cultured cell lines with the RNA Easy^{®} kit (Qiagen) according to the manufacturer's instructions. RNA concentrations were measured and 1 µg total cellular RNA was reverse transcribed in a 20 µl volume using oligo-(dT) as a primer and Moloney murine leukaemia virus reverse transcriptase (Pharmacia LKB Biotechnology, Milton Keynes, U.K.). A cDNA equivalent of 1ng RNA was amplified by the polymerase chain reaction using primers specific for the target genes. PCR was performed in a 50 µl reaction mixture with 250µM of each dNTP, 100nM of primers, 5µl of 10x buffer (HT Biotechnology Ltd, Cambridge, U.K.), and 1 unit of super Taq DNA polymerase (HT Biotechnology Ltd, Cambridge, U.K.), using 30 cycles. The reaction mix (25µl samples) was analyzed by agarose gel electrophoresis (1%) in TAE buffer containing 0.2µg/ml ethidium bromide. In all experiments, a mock PCR (without added DNA) was performed to exclude contamination. To exclude carry over of genomic DNA during the RNA preparation step, controls were also carried out in which the reverse transcriptase enzyme was omitted.

### Cell Transfection and FMG-mediated Cell Killing Assays

To assess the cytotoxicity of the GALV FMG driven by the HSE-Tyr-300, Tyr-300 or the CMV promoters, cells were plated in 6 well plates at a density of 5x10⁵ cells per well. 24 hours, later, each well was transfected with 5µg of the appropriate plasmid DNA using calcium phosphate transfection (Profection^{®}, Promega, WI) according to the manufacturer's instructions. 4 hours later the cells were washed three times in serum free medium and then incubated in normal, serum containing medium for a further 72 hours. Plates were either counted for living cells to assess the levels of cell survival (as described in Bateman, et al., *supra*) or were stained with Crystal violet to obtain a qualitative, pictorial representation, of the degree of cytotoxicity. To determine the heat shock sensitivity of the HSE element, the appropriate plates were sublethally heat shocked at 42°C for 30 minutes.

### Generation of Retroviral Vector Stocks

The GALV FMG cDNA was subcloned into the pBabePuro vector backbone at the *EcoR1* sites in the polylinker using standard techniques. Subsequently, the cDNA for the HSF-1d202-316 transcription factor (Zuo, et al., *supra)* was subcloned downstream of the GALV cDNA into the *Sal1* site of the pBabePuro polylinker leaving a 24 base pair linker to separate the GALV and HSF-1d202-316 genes. This C-type vector was packaged into viral particles by transfection of 5 µg of plasmid DNA into the 293INT cell line which stably expresses the MoMLV *gag* and *pol* genes but no envelope along with 5µg of the plasmid pMD.G encoding the VSV-G envelope protein (see, Naldini, et al., Science 272: 263-266 (1996); Zufferey, et al., Nat Biotechnol. 15: 871-875 (1997)).

Transfections were carried out in a 10 cm plate out using the Profection (calcium phosphate co-precipitation) method (Promega, WI). 48-72 hours following transfection, cell supernatants were recovered, filtered through a 0.45 µm filter and either used directly for infection or frozen at -80°C.

Viral infections were performed by exposing exponentially growing target cells, (either melanoma, Me1624 or MeWo) or non-melanoma (HT1080, 293 or TelCeB6), to different dilutions of viral supernatants in serum free medium for 4 hours in 24 well plates. The infectious medium was then removed and cells were washed three times before being allowed to grow in normal growth medium for a further 120 hours. At this stage, infected cultures were counted for living cells to assess the levels of cell survival.

### EXAMPLE 1

### Identification Of A Highly Melanoma Specific 300 Hi, Element Of A Human Tyrosinase Promoter

The specificity of enhancer + promoter elements for expression of a CAT reporter gene was compared in human melanoma cell lines (B 16, A378M) and human non-melanoma cell lines (HT1O8O, 293, Tel.CeB6). Plasmid construction and transfection were as described in Diaz et al., *supra.* Reverse transcriptase PCR was performed as described in Vile et al., *supra.*

The results of RT-PCR analysis are shown in Figure 2. The TDE-SV40 element is clearly preferentially active in melanoma cells, but this specificity is not sufficient to prevent a highly potent gene such as GALV from being toxic in non-melanoma cells.

Different lengths of the basal promoter from the human tyrosinase gene were surveyed in search of a promoter which is genuinely transcriptionally silent in non-melanoma cells. Using a sensitive nested RT-PCR assay with chloramphenicol acetyltransferase (CAT) as the reporter gene (the primers used were 5'-ATGGAGAAAAAAATCACTGGA-3' (SEQ ID NO:3) and 5'-GAGACGAAAAACATATTCTCA-3' (SEQ ID NO:4)), a 115 base pair element that was previously reported to be melanoma -pecific (Bentley, et al., *supra*) was found to possess transcriptional activity in some of the non-melanoma cells tested (Figure 3A). However, a 300 base pair element (Tyr300) was transcriptionally silent in all of the non-melanoma cell lines tested, yet retained activity in human and murine melanoma cells (Figure 3B).

### EXAMPLE 2

### HSE Adds Inducibility By Heat Shock Proteins To A Tyr300-SV4O Promoter

A consensus HSE sequence (5'-AGAATGTTCTAGAAG-3', SEQ ID NO:2) was synthesized and incorporated upstream of the melanoma-specific Tyr300 promoter element controlling a reporter gene encoding GC-CSF. This HSE consensus sequence confers heat shock inducibility on reporter genes (Zuo et al, *supra;* Goldenberg et al., J. Biol. Chem. 263: 19734-39 (1988)). Two plasmids were made in which HSE was separated from the Tyr300 promoter by either one full turn (10 nucleotide bases) of the DNA helix (HSE-Tyr300-FULL-GM-CSF) or by one-half turn of the helix (HSE-Tyr300-HALF-GM-CSF).

Transient transfection of a human non-melanoma line (HT1080) with 20 ug of either a Tyr-300-GM-CSF plasmid, an HSE-Tyr 300-FULL-GM-CSF plasmid, or an HSE-Tyr 300-HALF-GM-CSF plasmid generated no detectable GM-CSF product 72 hrs following transfection (data not shown). Co-transfection of these same cells with HSF-1 cDNA also failed to produce any GM-CSF production (see Figure 4, condition 1). Human melanoma cells (MeWo cell line) transfected with 20 ug of either the Tyr 300-GM-CSF (Figure 4, condition 2) or the HSE-Tyr 300-FULL-GM-CSF plasmid (Figure 4, condition 6) express only very low amounts of GM-CSF; similar results were obtained with the HSE-Tyr 300-HALF-GM-CSF plasmid (not shown).

This demonstrates that Tyr 300 is a very weak promoter. However, transfection of MeWo cells with 20 µg of the TDE-SV4O-GM-CSF plasmid (Figure 4, condition 3) leads to easily detectable levels of GM-CSF production (see also Diaz et al., J. Virol. (1998) 72:789-95). In the presence of either heat shock (42 °C, 30 minutes; Figure 4, condition 4) or co-transfection with 20 µg of mHSF-1 plasmid (Figure 4, condition 5), GM-CSF production is increased significantly following transfection with the HSE-Tyr 300-FULL-GM-CSF plasmid and increased to a lesser degree using the HSE-Tyr300-HALF-GM-CSF plasmid (not shown). These experiments demonstrate that the HSE-Tyr300 base pair promoter is both tissue-specific and can be induced by mHSF-1.

### EXAMPLE 3

### Induction Of Hsp70 Using HSE And Constitutively Active Mutant HSF-L.

Hsp70 expression was probed by Western blotting using antibody BRM-22 (Sigma Chemical Co., St. Louis, MO) in B16 cells stably expressing constitutively active human mHSF-1 (deletion 202-3 16). The resulting immunoblot is shown in Figure 5. Lysates of untransfected B-16 cells are shown in lane 1. Lane 2 shows the pooled population of HSF-1 transfected colonies. Lanes 3-7 show clones of individual HSF-1 transfected colonies. Mock transfected cells or cells transfected with irrelevant plasmids did not show induction of hsp70. Expression of hsp70 correlated exactly with expression of mHSF-1 assayed by Western Blotting of the pools and clones (data not shown).

### EXAMPLE 4

### Selective Elimination Of Human Melanoma Tumor Growth Using A Tyr300-GALV Construct

HT1080 (human fibrosarcoma) or Mel624 (human melanoma) tumors were seeded subcutaneously in nude mice (10⁶ cells per mouse). Growing tumor cells were transduced *in situ* with 10 ug/tumor of CMV-β-Gal, CMV-GALV or Tyr300-GALV plasmid DNA complexed with Efectene lipid (Qiagen). The CMV β-Gal plasmid was constructed by cloning β-galactosidase cDNA into the EcoR1 site of the plasmid pCR3 (Invitrogen) with expression driven by the CMV promoter. The CMV-GALV plasmid was constructed by cloning GALV cDNA into the EcoRl site of pCR3 with expression driven by the CMV promoter. The Tyr300-GALV plasmid was constructed by cloning the HSE-Tyr300 element upstream of the GALV cDNA into the EcoRl site of pCR3; for this condition the cells were cotransfected with mHSF-1 in pCR3: Ten mice were injected per group. At seven days following DNA injection of HT1080 tumors, those injected with CMV-GALV plasmid began to regress compared with the progression of tumors in the other two groups (Figure 7A). In the same mice at 13 days after transduction, tumor size in some groups reached 1.2 cm in the longest diameter, at which point those animals were sacrificed. The tumors in the CMV-GALV-injected group had all regressed and had been eliminated (Figure 7B). Animals in which tumor size had not reached 1.2 cm by day 13 were maintained and tumor size was followed.

By day 30 following transduction of tumors, nearly all mice in the CMV-β-Gal and TYR-GALV groups had eventually developed tumors which reached 1.2 cm in diameter and were sacrificed (Figure 7C). Long term tumor-free mice (shown over each group) were scored as those having no detectable tumor by the end of the experiment. Progression of transduced human melanoma tumors, Mel624, was similar to that shown for the HT1080 tumors. By the end of the experiment (day 30 after transduction), 90% the tumors transduced with the CMV-GALV plasmid had been eliminated. The one tumor recurrence in this group developed 23 days following DNA delivery. In contrast, 100% of the tumors transduced with the TYR-GALV plasmids were eliminated and no regrowths were observed.

### Example 5

### The Efficacy Of Transcriptional Targeting Depends On The Potency Of The Transgene Being Expressed

In one embodiment, the cDNA of the GALV FMG protein was cloned downstream of the TDE-SV4O element to induce melanoma-specific cell killing *in vitro.* Transfection of the TDE-SV4O-GALV plasmid into three different human melanoma cell lines induced large amounts of cell fusion 24-48 hours following transfection, at levels comparable to that produced by a CMV-GALV construct. When transfected into 5 human non-melanoma cell lines, the TDE-SV4O-GALV construct showed a clear lag period in the formation of syncytia compared to CMV-GALV. However, in the majority of the lines, significant amounts of cell fusion subsequently developed after 72-96 hours as shown in Table 1, below.

| | Syncytium Formation 24 hrs Post Transfection | |
|---|---|---|
| Cell Line | CMV GALV | TDESV40 GALV |
| HT1080 | +++ | +/- |
| 293 | +++ | + |
| Tel | +++ | +/- |
| Vero | +++ | - |
| MeWo | +++ | +++ |
| A378M | +++ | +++ |
| | Syncytium Formation 96 Hours Post Transfection | |
| Cell Line | CMV-GALV | TDESV40-GALV |
| HT1080 | +++ | ++ |
| 293 | +++ | +++ |
| Tel | +++ | ++ |
| Vero | +++ | + |
| MeWo | +++ | +++ |
| A378M | +++ | +++ |

| | | |
|---|---|---|
| +++: 70-100% of all the cellls on the plate have been recruited into syncytia; ++: 30-70% of cells are within syncytia; +: between 10 and 30% are within syncytia; and +/-: means that fewer than 10% of cells are within syncytia. | | |

To confirm that expression from the TDE-SV4O enhancer/promoter is genuinely melanoma-preferential, a semi-quantitative RT-PCR assay was used (Figure 8). Levels of transcripts from the TDE-SV40 promoter were significantly lower than those from the CMV driven construct in all non-melanoma cell lines, but were close to equivalent in the melanoma cell lines (Figure 8). Thus, the melanoma cell specificity of TDE-SV40 is real but is not sufficient to prevent a highly potent gene such as GALV from being toxic in a proportion of non-melanoma cells.

### Example 6

### Identification Of An Element Of The Human Tyrosinase Promoter That Is Transcriptionally Silent In Non-Melanoma Cells But Retains Activity In Melanoma Cell Lines

In one embodiment, fragments which ranged from +80 to -65, -115 or -300 from this basal Tyr promoter were evaluated using both CAT expression and RT-PCR assays to screen for expression. Both the 65 base pair and the 115 base pair fragments of the promoter showed some transcriptional activity by RT-PCR in some, or all, of the non-melanoma cells tested (Figure 9A). In contrast, using a sensitive nested RT-PCR assay, the 300 base pair element of the tyrosinase promoter was transcriptionally silent in all of the non-melanoma cells tested but retained activity in the human and murine melanoma cells (Figure 9B). However, when the CAT, GM-CSF, or GALV genes were cloned downstream of the 300 base pair promoter, levels of transgene expression were low, and melanoma cells transfected with the Tyr 300-GALV construct showed limited cell cytotoxicity at only about 10% of the levels produced by CMV-GALV (Figure 14).

### Example 7

### Amplification Of Low Level Expression From A Weak, But Tissue-Specific, Promoter Using A Second Transcriptional Regulatory Element

Since levels of expression from highly cell type-specific promoters are generally not therapeutic, even when highly potent genes (e.g., cytotoxic) genes such as FMGs are used, in one embodiment, an amplification promoter element was operably linked to an FMG under the control of a highly cell type-specific promoter. In one embodiment, an amplification promoter comprising a consensus HSE sequence ( 5'- AGAATGTTCTAGAAG-3') modified from the construct described in Goldenberg, et al., *supra*, and Todyry, et al., *supra,* was synthesized

In order to investigate whether it was necessary to optimize the topological spacing of the HSE element relative to any of the 5 characterized important DNA/protein binding sites within the 300 base pair element of the tyrosinase promoter, plasmids were made in which the HSE element was separated from the C nucleotide at position -300 of the Tyr-300 promoter by either no nucleotides or one full turn of the DNA helix (HSE-Tyr 300-FULL) or by a stuffer fragment representing one half turn of the helix (HSE Tyr-300-HALF) (Figure 9A). Both HSE-Tyr 300-GM-CSF plasmids transfected into MeWo melanoma cells produced the same low levels of GM-CSF as the Tyr300-GM-CSF plasmid (Figure 9B). However, when the transfected cells were heat shocked at 42°C for 30 minutes, 24 hours following transfection, GM-CSF production was increased, but only in cells transfected with the HSE-Tyr300 plasmids (Figure 9B).

Several experiments demonstrated up to a three fold increase in GM-CSF production from melanoma cells transfected with the HSE-Tyr 300-FULL construct compared to the HSE-Tyr 300-HALF construct (data not shown). The non-melanoma line HT1080, similarly treated, did not produce any GM-CSF either with or without heat shock *in vitro* (data not shown). Similar results were obtained from RT-PCR studies performed on melanoma and non-melanoma cells transfected with the HSE-Tyr 300-FULL or HSE-Tyr 300-HALF constructs, confirming both the tissue specificity of GM-CSF expression from melanoma cells, as well as the increased level of message using the HSE-Tyr 300-FULL construct (data not shown). Hence, the HSE element cooperates with the Tyr300 base pair promoter to induce tissue-specific expression, an effect which can be optimized by engineering the topology of the spacing of the two separate elements.]

### Example 8

### Transcriptional Transactivation Of The HSE-Tyr300 Base Pair Tissue-Specific Promoter Is Possible Using Mutant HSF-1

When the HSE-Tyr-300-FULL element was used to express the GALV FMG, transfection was unable to eradicate melanoma cells *in vitro* to the same extent as the CMV promoter (Figure 14). Therefore, in one embodiment, constitutively active forms of HSF-1 were used to increase levels of expression from the tissue specific HSE-Tyr300-FULL element.

A deleted, mutant form of HSF-1, HSF-1d202-316, was used in which deletion of amino acids 202-316 removes the sensitivity to stress dependent activation and nuclear translocation. The protein is constitutively active in the absence of cellular stress while retaining the DNA and protein binding domains required to transactivate gene expression through HSE. Co-expression of HSF- 1d202-316 was demonstrated to transactivate the very weak, but highly specific, HSE-Tyr 300 promoter (Figure 10). When HSF1d202-316 was co-transfected into MeWo melanoma cells along with the Tyr 300-GM-CSF construct, no GM-CSF production could be detected. However, when HSF-1d202-316 was co-transfected with the HSE-Tyr 300-FULL-GM-CSF construct, levels of GM-CSF were increased significantly to a level similar to that produced by heat shock (Figure 9B). Importantly, cotransfection of HSE-Tyr-300-GM-CSF with HSF-1d202-316 into non-melanoma cells still gave no detectable GM-CSF production (Figure 9B). Therefore, even low levels of HSF-1d202-316 (as provided in a transient co-transfection assay) were capable of transactivating the HSE-Tyr300 base pair promoter element while retaining the tissue specificity of the tyrosinase promoter.

### Example 9

### The HSE Transcriptional Control Element Can Be Used To Transactivate Gene Expression From The Melanoma-Specific Tyr-300 Promoter

In one embodiment, the HSE-Tyr 300 transcriptional regulatory element was used in tandem with the HSF-1d202-316 transcription factor, to regulate highly tissue-specific expression even of a very potent cytotoxic genes (Figure 11) and to provide an immunostimulatory effect.

In one embodiment, murine B16 cells were co-transfected with HSE-Tyr-300-GALV and HSF-1d202-316 plasmids. Importantly, B16 cells are not fused by expression of the GALV FMG because they lack the Pit-1 receptor. RT-PCR was used to follow expression of the transgenes (Figure 12). The co-transfected HSF-1d202-316 was expressed within 24 hours of transfection. However, the GALV transgene expressed from the HSE-Tyr element was not detected in appreciable amounts until 72 hrs following transfection (Figure 12), presumably because sufficient levels of HSF-1d202-316 were required to build up in order to transactivate the HSE element. In addition, following appearance of mRNA for the transfected HSF-1d202-316, transactivation of endogenous hsp70 was detected 24 hrs later. These data confirm both the operation of the feedback loop at the transcriptional level and the induction of endogenous heat shock and stress response genes. The latter induction provides effective adjuvant functions for immunostimulation.

### Example 10

### The HSE-Tyr-300/HSF-1 Feedback Loop Can Be Used To Kill Melanoma Cells Specifically And Efficiently

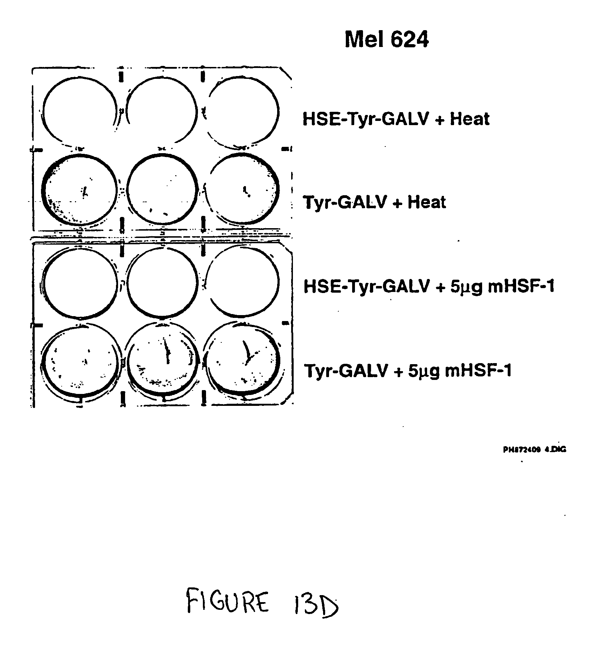

To test the efficacy of the transcriptional feedback loop *in vitro,* the HSE-Tyr300 GALV construct was transfected into non-melanoma human cells, Tel.CeB6 (Figures 13A and B) or HT1080 (data not shown). The only significant toxicity was seen in the cells transfected with the CMV-GALV construct (Figure 13A). In contrast, both the HSE-Tyr-300 and Tyr-300-GALV constructs gave low levels of toxicity when transfected into a melanoma line, Me1624 (Figures 13C, 13D) or similarly the MeWo line (data not shown). Quantitation of cell survival showed that the HSE-Tyr-300 construct gave small but significantly enhanced killing of Mel624 cells with respect to the Tyr-300 construct, presumably due to the proposed activation of the HSE-Tyr-300 element through induction of endogenous HSF-1 through GALV-mediated cell killing.

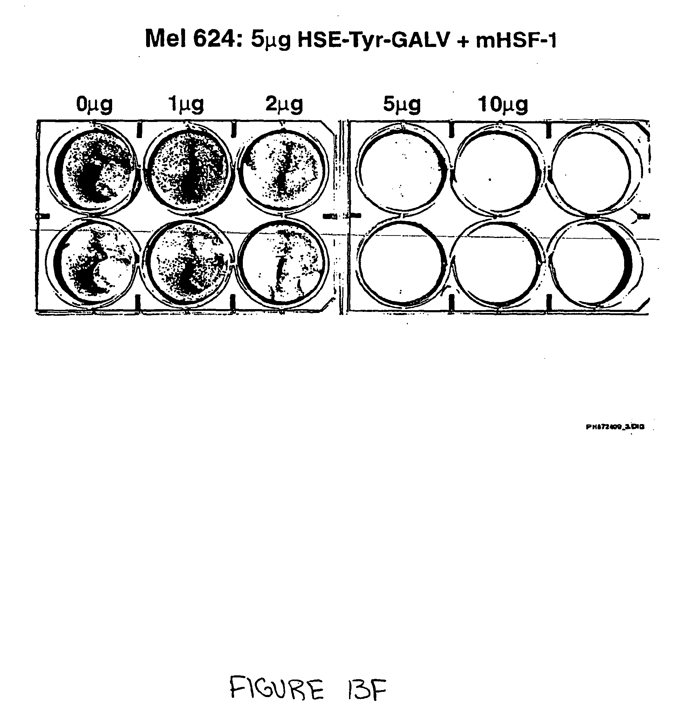

However, greatly enhanced toxicity was observed with transfection of the HSE-Tyr-300-GALV constructs when the cells were either heat shocked or co-transfected with HSF-1d202-316 (Figure 13C, D). That the latter effects were operative through HSF-1d202-316 regulation were confirmed by the demonstration that increasing amounts of co-transfected HSF-id202-316 led to proportional increases in killing of melanoma cells, whereas no killing was observed with increasing concentrations of an irrelevant co-transfected β-Gal plasmid (Figures 13E, F). Taken together, these data show that it is possible to control highly tissue-specific expression of GALV FMG either through the application of heat itself, enabling locoregional control of gene expression (see, e.g., Blackburn, et al., Cancer Res. 58: 1358-1362, (1998) ), or through co-expression of the HSF-1d202-316 transcription factor.

### Example 11

### Expression Of HSF-1d202-316 Induces Hsp70 And Other Stress Related Proteins

As well as leading to direct cytotoxicity through FMG-mediated fusion, the presence of the HSF1d202-316 transcription factor leads to activation of endogenous HSEs upstream of stress response genes, including hsp70 (Baler, et al., Mol Cell Biol; 13: 2486-2496 (1993); Zuo, et al., *supra*). Induction of hsp70 is potently immunostimulatory, but optimally so, if it occurs during cell killing *in vivo.* Expression of HSF-1 is immunotherapeutic, through activation of endogenous HSE elements. Transfection of HSF-1d202-316 induces expression of hsp70 mRNA (see Figure 12) and induction can be detected at the level of protein expression in a variety of human and murine cell lines (data not shown). HSF-1d202-316 additionally activates expression of other stress-related proteins which also enhance the immunogenicity of the dying tumor cells, such as MICB, a ligand for the NK activatory receptor NKG2D (data not shown).

### Example 12

### The Complete HSE-Tyr300-FMG-HSF-1 Feedback Loop Can Be Incorporated Within The Context Of A Hybrid LTR Retroviral Vector

In one embodiment, the HSE-Tyr300 element was cloned into the 3'LTR of the Mo-MLV derived retroviral vector pBabe Puro (Morgenstern, et al., *supra).* The HSE-Tyr300 enhancer/promoter was placed in the 1J3 region of the viral LTR as described in Diaz, et al., *supra,* replacing the viral enhancer and promoter regions of the U3 region of the LTR with the heterologous enhancer/promoter elements (HSE-Tyr-300). Simultaneously, the GALV-FMG cDNA was cloned into the polylinker of the vector. As an added strategy to decrease the chances of initiating the feedback loop in non-melanoma cells, the HSF-1d202-316 cDNA was cloned downstream of the GALV cDNA, separated from it by a linker of 24 base pair (Figure 14A), thereby exploiting a strategy as described by Cosset, et al., *supra.*

The lack of an internal ribosome entry site (IRES) between the first (GALV) and the second (HSF-1d202-316) gene in this construct means that only a small proportion of the total mRNA molecules produced from the HSE-Tyr-300 promoter will undergo internal initiation of translation of the second transgene (Cosset, et al., *supra).* Hence, only those cells in which the promoter is sufficiently active will be able to generate enough mRNA such that any of the second gene is ever translated. Therefore, any non-melanoma cell, in which there is appreciable leakiness of the Tyr-300 element, should still have very low levels of expression of the feedback gene (HSF-1d202-316), significantly reducing the chances of the feedback loop ever being initiated.

Following packaging, reverse transcription, and integration of the retroviral vector, an integrated provirus of structure shown in Figure 14A was produced. Virus was packaged from the 293INT cell line with the VSV-G envelope and used to infect MeWo or Me1624 melanoma and HT1080, 293 or Tel non-melanoma cell lines. The ability of different packaged viral vectors to induce syncytia and cytotoxicity was followed with time (Figures 14B, 14C). Whereas the control vector pBabe-Puro generated no detectable syncytia in either melanoma or non-melanoma cell lines, the positive control vectors pBabe-GALV and pBabe GALV-HSF-1 (wt LTR), in which the GALV cDNA is driven by the Mo-MLV LTR, both induced syncytial formation within 24 hours of infection of all cell types. This led to extensive cell killing over time.

In contrast, the pBabe-GALV-HSF-1 (HSE-Tyr LTR) vector produced no detectable syncytia in either of the three non-melanoma cell lines, indicating that the LTR was unable to drive expression of the GALV cDNA. Further no vector-induced syncytia were observed in infected 293 cells. These cells express the adenoviral E1A gene, which transactivate expression of cellular hsp70 through the HSE element. Despite constitutive transactivation of the HSE element, the tissue specificity of the TYR-300 element was tight enough to prevent initiation of the feedback loop in these cells. However, in the two melanoma cell lines tested, syncytia were induced, but only 48-72 hours following the appearance of syncytia with the wild type LTR. Presumably this is due to the time lag required for the build up to sufficient levels of HSF-1d202-316 protein to transactivate the HSE element. Thereafter, syncytial development was very aggressive and the pBabe-GALV-HSF-1 (HSE-Tyr LTR) was able to kill over 90% of target melanoma cells, comparable to that of the wild type LTR-driven GALV vector. Finally, to confirm that longer periods of culture did not allow pBabe-GALV-HSF-1 (HSE-Tyr LTR) to induce syncytia, infected TelCeB6 cells were passaged for another week and inspected daily for syncytia. None were detected. Therefore, although there is a clear time delay in the ability of the pBabe-GALV-HSF-1 (HSE-Tyr LTR) vector to become effective, the vector is both highly tissue-specific and effective.

## Claims

1. A composition comprising a nucleic acid, wherein said nucleic acid comprises:
(a) a cell type-specific promoter for activating the expression of a gene in a specific cell type, wherein the cell type-specific promoter is human Tyr300, defined by SEQ ID NO 1
(b) a therapeutic gene sequence operably linked to said cell type-specific promoter;
(c) an amplification promoter element for amplifying transcription of said therapeutic gene in said specific cell type, wherein said amplification promoter element is a heat shock element (HSE); and
(d) a sequence encoding a transcription activator, said transcription activator for activating said amplification promoter element, and wherein said transcription activator is heat shock factor-1 (HSF-1).

2. The composition of claim 1, wherein said sequence encoding said transcription activator and said therapeutic gene sequence are on different nucleic acid molecules.

3. The composition of claim 1, wherein said amplification promoter element comprises at least one human HSE consensus sequence.

4. The composition of claim 1, wherein said therapeutic gene is a cytotoxic gene.

5. The composition of claim 4, wherein said cytotoxic gene encodes a fusogenic protein.

6. The composition of claim 4, wherein the cytotoxic gene encodes GALVenv, HSVTK, cytosine deaminase, nitroreductase, or VSV-G glycoprotein.

7. The composition of claim 1, wherein said transcription activator is constitutively expressed.

8. The composition of claim 1, wherein said therapeutic gene sequence and the sequence encoding said transcription activator are both operably linked to said cell type-specific promoter.

9. The composition of claim 1, wherein said therapeutic gene sequence and the sequence encoding said transcription activator are both operably linked to said amplification promoter element.

10. The composition of claim 1, wherein said transcription activator is HSF-1 which lacks amino acid residues 202-316.

11. The composition according to any of claims 1 to 10 for use as a medicament.

## Patentansprüche

1. Zusammensetzung umfassend eine Nukleinsäure, wobei die Nukleinsäure umfasst:
(a) einen Zelltyp-spezifischen Promotor zum Aktivieren der Expression eines Gens in einem spezifischen Zelltyp, wobei der Zelltyp-spezifische Promotor ein durch SEQ ID NR. 1 definierter humaner Tyr300 ist;
(b) eine mit dem Zelltyp-spezifischen Promotor funktionell verknüpfte Sequenz eines therapeutischen Gens;
(c) ein Verstärkungspromotorelement zum Verstärken der Transkription des therapeutischen Gens im spezifischen Zelltyp, wobei das Verstärkungspromotorelement ein Hitzeschockelement (HSE) ist; und
(d) eine einen Transkriptionsaktivator kodierende Sequenz, wobei der Transkriptionsaktivator zum Aktivieren des Verstärkungspromotorelements ist und wobei der Transkriptionsaktivator Hitzeschockfaktor-1 (HSF-1) ist.

2. Zusammensetzung nach Anspruch 1, wobei die den Transkriptionsaktivator kodierende Sequenz und die Sequenz des therapeutischen Gens auf verschiedenen Nukleinsäuremolekülen sind.

3. Zusammensetzung nach Anspruch 1, wobei das Verstärkungspromotorelement zumindest eine humane HSE-Konsensussequenz umfasst.

4. Zusammensetzung nach Anspruch 1, wobei das therapeutische Gen ein zytotoxisches Gen ist.

5. Zusammensetzung nach Anspruch 4, wobei das zytotoxische Gen ein fusogenes Protein kodiert.

6. Zusammensetzung nach Anspruch 4, wobei das zytotoxische Gen GALVenv, HSVTK, Cytosin-Deaminase, Nitroreduktase oder VSV-G-Glycoprotein kodiert.

7. Zusammensetzung nach Anspruch 1, wobei der Transkriptionsaktivator konstitutiv exprimiert wird.

8. Zusammensetzung nach Anspruch 1, wobei sowohl die Sequenz des therapeutischen Gens als auch die den Transkriptionsaktivator kodierende Sequenz mit dem Zelltyp-spezifischen Promotor funktionell verknüpft sind.

9. Zusammensetzung nach Anspruch 1, wobei sowohl die Sequenz des therapeutischen Gens als auch die den Transkriptionsaktivator kodierende Sequenz mit dem Verstärkungspromotorelement funktionell verknüpft sind.

10. Zusammensetzung nach Anspruch 1, wobei der Transkriptionsaktivator ein HSF-1 ist, welchem Aminosäurereste 202-316 fehlen.

11. Zusammensetzung nach einem der Ansprüche 1 bis 10 zur Verwendung als ein Medikament.

## Revendications

1. Composition comprenant un acide nucléique, dans laquelle ledit acide nucléique comprend :
(a) un promoteur spécifique à un type de cellule destiné à activer l'expression d'un gène dans un type de cellule spécifique, où le promoteur spécifique à un type de cellule est une séquence Tyr300 humaine définie par le numéro d'identification de séquence 1,
(b) une séquence de gène thérapeutique liée fonctionnellement audit promoteur spécifique à un type de cellule,
(c) un élément de promoteur d'amplification destiné à amplifier une transcription dudit gène thérapeutique dans ledit type de cellule spécifique, où ledit élément de promoteur d'amplification est un élément de choc thermique (HSE), et
(d) une séquence codant un activateur de transcription, ledit activateur de transcription étant destiné à activer ledit élément de promoteur d'amplification, et où ledit activateur de transcription est un facteur de choc thermique 1 (HSF-1).

2. Composition selon la revendication 1, dans laquelle ladite séquence codant ledit activateur de transcription et ladite séquence de gène thérapeutique sont sur des molécules d'acides nucléiques différentes.

3. Composition selon la revendication 1, dans laquelle ledit élément de promoteur d'amplification comprend au moins une séquence consensus HSE humaine.

4. Composition selon la revendication 1, dans laquelle ledit gène thérapeutique est un gène cytotoxique.

5. Composition selon la revendication 4, dans laquelle ledit gène cytotoxique code une protéine fusogène.

6. Composition selon la revendication 4, dans laquelle le gène cytotoxique code GALVenv, HSVTK, une désaminase de cytosine, une nitroréductase, ou une glycoprotéine VSV-G.

7. Composition selon la revendication 1, dans laquelle ledit activateur de transcription est exprimé de manière constitutive.

8. Composition selon la revendication 1, dans laquelle ladite séquence de gène thérapeutique et la séquence codant ledit activateur de transcription sont toutes deux liées fonctionnellement audit promoteur spécifique à un type de cellule.

9. Composition selon la revendication 1, dans laquelle ladite séquence de gène thérapeutique et la séquence codant ledit activateur de transcription sont toutes deux liées fonctionnellement audit élément de promoteur d'amplification.

10. Composition selon la revendication 1, dans laquelle ledit activateur de transcription est un facteur HSF-1 auquel manquent les résidus d'acides aminés 202 à 316.

11. Composition selon l'une quelconque des revendications 1 à 10, destinée à être utilisée en tant que médicament.
